# EUROPEAN PATENT SPECIFICATION

(11) **EP 1 458 797 B1**
(45) Date of publication and mention of the grant of the patent: **23.12.2009**
(21) Application number: 02793969.3
(22) Date of filing: 20.11.2002
(51) Int. Cl.: C08J 9/24

(54) **DISCRETE HYDROPHILIC-HYDROPHOBIC POROUS MATERIALS AND METHODS FOR MAKING THE SAME**
DISKRETE HYDROPHIL-HYDROPHOBE PORÖSE MATERIALIEN UND HERSTELLUNGSVERFAHREN DAFÜR
MATERIAUX POREUX A PROPRIETES HYDROPHILES ET HYDROPHOBES DISTINCTES ET PROCEDES DE FABRICATION

(30) Priority: 21.11.2001 US 331723 P
(43) Date of publication of application: 22.09.2004
(73) Proprietor: Porex Corporation, College Park, GA 30349 (US)
(72) Inventor: MAO, Guoqiang, Smyrna, GA 30082 (US); GREENE, George, W., IV, Goleta, CA 93117, USA (US); COPPOLA, Richard, J., Peachtree City, GA 30269 (US); YAO, Li, Danville, CA 94526 (US)
(74) Representative: Dey, Michael
(86) International application number: PCT/US2002/037224
(87) International publication number: WO 2003/046062

(56) References cited:
- EP-A- 0 437 721
- WO-A-98/32790
- WO-A-98/47701
- GB-A- 1 597 455
- US-A- 4 943 471
- US-A- 5 834 583
- US-A- 5 912 278

## Description

### 1. FIELD OF THE INVENTION

This invention relates to porous polymeric materials having a plurality of discrete surface regions of different properties, and methods for making the same.

### 2. BACKGROUND OF THE INVENTION

Porous materials, including metal, ceramic, glass and polymeric materials, have increasingly been used in a variety of applications, such as filtration, aeration, wicking, implant and other biomedical devices. For example, porous polymeric materials can be used in medical devices that serve as substitute blood vessels, synthetic and intra-ocular lenses, electrodes, catheters, and extra-corporeal devices such as those that are connected to the body to assist in surgery or dialysis. Porous polymeric materials can also be used as filters for the separation of blood into component blood cells and plasma, microfilters for removal of microorganisms from blood, and coatings for ophthalmic lenses to prevent endothelial damage upon implantation. Porous materials have also been used in diagnostic devices such as lateral flow devices, flow through devices and other immunoassay devices.

The hydrophobic nature of many polymers, however, has limited the usefulness of porous materials made from them. Therefore, attempts have been made to modify the surface properties of the porous materials. Various methods were contemplated to achieve such modifications on the surface properties. Examples of such methods include: addition of organic surfactants; plasma discharge as disclosed in, for example, U.S. Patent Nos. 4,845,132 and 6,022,902; electron beam discharge as disclosed in, for example, U.S. Patent No. 6,271,273; corona discharge as disclosed in, for example, U.S. Patent No. 5,688,465; and treatment by solutions as disclosed in, for example, U.S. Patent Nos. 5,540,837, 5,695,640, 5,700,559, 5,807,636, 5,837,377, 5,914,182, 5,916,585, and 6,060,410. The contents of the recited U.S. patents are incorporated herein by reference.

Among the different techniques available for modifying the surface property of polymeric materials, most are limited in their ability to control the degree to which a surface is modified, and many are expensive, inefficient, or unable to be used to modify porous surfaces without clogging their pores. Moreover, although different modifications can be introduced onto porous materials using these techniques, these techniques are directed to methods of modifying the surface properties of the whole piece of porous material.

On the other hand, porous materials with selectively predetermined discrete areas of various surface properties have a wide variety of applications in single piece diagnostic devices, multi-channel biological assays, microfluidic devices, combinatory chemistries, and fast biological molecule and drug screening. Therefore, there exists a need to develop a porous material with selectively predetermined discrete regions of various surface properties.

### 3. SUMMARY OF THE INVENTION

This invention encompasses novel porous materials containing discrete regions of different surface properties and methods of their manufacture. Materials of the invention comprise a porous substrate to which chemical or biological moieties are selectively bound in discrete patterns, or in which discrete regions on the surface of the substrate are selectively activated.

A first embodiment of this invention is directed to a single piece porous polymeric material having a surface that contains a plurality of discrete regions that exhibit distinct surface properties.

Specific examples of polymers from which materials of the present invention can be made include, but are not limited to, polyolefin, polyether, polyurethane, polycarbonate, polyetheretherketone, poly(phenylene oxide), poly(ether sulfone), polysulfone, nitrocellulose, cellulose, fluorinated polymers, PTFE, porous fiber materials or nylon. Polyolefins include, but are not limited to, ethylene vinyl acetate, ethylene methyl acrylate, polyethylene, polypropylene, ethylene-propylene rubber, ethylene-propylene-diene rubbers, poly(1-butene), polystyrene, poly(2-butene), poly(1-pentene), poly(2-pentene), poly(3-methyl-1-pentene), poly(4-methyl-1-pentene), 1,2-poly-1,3-butadiene, 1,4-poly-1,3-butadiene; polyisoprene, polychloroprene, poly(vinyl acetate), poly(vinylidene chloride), poly(vinylidene fluoride), poly(tetra fluoro ethylene), or mixtures thereof.

Surface properties that can be introduced onto the porous materials of this invention include, but are not limited to, hydrophilicity, hydrophobicity, oleophobicity, biological molecules binding capability, wetting or wicking property, presence or density of functional groups, chemical reactivity, electric charges, porosity, and pore sizes.

Discrete regions can consist of hydrophilic and hydrophobic regions. In this respect, the present invention encompasses a porous material wherein discrete hydrophilic regions are surrounded by hydrophobic boundaries. Alternatively, discrete hydrophobic regions may be surrounded by hydrophilic boundaries. In another aspect, discrete regions can also be hydrophobic and oleophobic.

In a given piece of porous material of this invention, all of the discrete regions may exhibit the same surface property. On the other hand, different discrete regions may have different surface properties in a given piece of the porous material.

The porous materials of this invention can include the ones wherein the discrete regions are coated with coating materials that alter the surface properties of the discrete regions. The coating can be single layered, double layered, or optionally multiple layered. The coating layers are bound to the layer right underneath them by covalent bonds, electrostatic interactions, or combination thereof.

Suitable coating materials include, but are not limited to, polyelectrolyte, neutral polymer, small molecule, biomolecule, or combination thereof.

Specific polyelectrolytes include, but are not limited to, a phosphate, polyethyleneimide, poly(vinylimidazoline), quaterized polyacrylamide, carboxyl modified polyacrylamide, polyvinylpyridine, poly(vinylpyrrolidone), polyvinylamine, polyallylamine, chitosan, polylysine, poly(acrylate trialkyl ammonia salt ester), cellulose, poly(acrylic acid), polymethylacrylic acid, poly(maleic acid), poly(styrenesulfuric acid), poly(vinylsulfonic acid), poly(toluene sulfuric acid), poly(methyl vinyl ether-alt-maleic acid), poly(glutamic acid), Nafion^{®} (DuPont), surfactant, dextran sulfate, hyaluric acid, heparin, alginic acid, adipic acid, chemical dye, protein, enzyme, nucleic acid, peptide, or a salt, ester, and/or copolymer thereof.

Specific neutral polymers include, but are not limited to, isocyannated terminated polymer, epoxy-terminated polymer, or hydroxylsuccimide terminated polymer. More specific examples of neutral polymer include polyurethane, poly(ethylene glycol), and polysiloxane.

Specific small molecules include, but are not limited to, sodium dodecylsulfonate, dodecyltrimethylamonium bromide, phosphate, sulfonate, bronate, sulfonate, dye, lipid, metal ion, or surfactant containing fluorine.

Specific biomolecules include, but are not limited to, proteins, enzymes, lipids, hormones, peptides, nucleic acids, oligonucleic acids, DNA, RNA, sugars, or polysaccharides.

The porous material of this invention may have electric charges in the discrete regions. The charge may be the same in all discrete regions, or different charges can be present in different discrete regions. In addition, regardless of whether all of the discrete regions have the same charge or not, different charge densities may be present in different discrete regions.

Moreover, the porous material of this invention may have chemical functional groups in the discrete regions. All of the discrete regions may contain the same chemical functional group, or different discrete regions may contain different chemical functional groups. Again, regardless of the type of functional groups, different discrete regions can contain different densities of chemical functional groups. Specific examples of chemical functional groups include, but are not limited to, an amino group, carboxylic acid, sulfuric acid, a polysaccharide, or a perfluroalkyl group.

In another aspect of this invention, the discrete regions on the material of this invention can be three dimensionally located. In other words, the porous discrete parts in the invention can also be in z-direction, in addition to x- and/or y-direction. For example, on a single piece of discrete porous material of this invention, upper part of the material may have a different chemical property from the bottom part.

In another embodiment, this invention is directed to a method of making porous materials having a plurality of discrete regions that exhibit distinct surface properties. The method comprises arranging polymer particles that have different properties in a predetermined pattern and compress sintering the particles to provide a porous material. In certain cases, the resulting porous material can take the form of a billet. In such cases, the porous material can optionally be skived into sheets.

In another embodiment, the porous materials of this invention are made by a method comprising selectively placing polymer powders in a predetermined pattern on discrete regions of a porous substrate and compress sintering the polymer powders into the substrate.

In one aspect, the same polymer powders are used in all discrete regions on the substrate to provide all discrete regions with the same chemical properties. As a variation, polymer powders made from different polymers can be used to provide discrete regions of different chemical surface properties in a given porous material.

In a specific embodiment, polymer powders that are hydrophilic are placed onto discrete regions of hydrophobic porous substrate. The resulting porous material has discrete hydrophilic-hydrophobic regions.

In one aspect, certain specific embodiments of this invention are directed to a method of making a porous material that exhibits the same porosity or pore size in all discrete regions. Alternatively, different porosity or pore sizes may be introduced onto different discrete regions.

Another embodiment of this invention is directed to another method of making the porous materials of this invention. This method comprises providing a porous substrate and selectively activating discrete regions of the substrate in a predetermined manner. To activate the surface of the porous substrate, high-energy activation is used. Examples of high-energy activation include, but are not limited to, direct and remote plasma activation, corona discharge, electron beam, and UV radiation. Surface regions that are exposed to the high energy activation acquire hydrophilic properties, whereas other regions of the surface remain hydrophobic.

Several different methods are employed to yield selective activation of discrete regions. For activation methods such as plasma activation, corona discharge, or UV radiation, one or more masks with desired pattern, which can shield certain portions of the substrate while leaving other portions exposed, can be employed to achieve selective activation. Suitable materials from which the mask can be made include, but are not limited to, metals, plastics, rubbers, and paper tapes.

In case that the corona discharge activation is used, a specially designed electrode can be used to achieve a selective activation in addition to the use of masks. Selective activation is achieved because the electrode is designed to have the exact shape of the discrete regions to be activated, thus the regions exposed to the electrode will become hydrophilic, while the unexposed regions will remain hydrophobic.

For activation methods such as electron beam activation, the electron beam can simply be focused to specified discrete regions to achieve the selective activation.

The porous materials of this invention may contain discrete regions that have been subjected to coating procedures. Accordingly, another embodiment of this invention is directed to a method comprising coating the discrete surface regions with coating materials for a time and at a temperature sufficient for a formation of a layer covering the discrete regions. The coating materials can include polyelectrolyte, neutral polymer, small molecule, biomolecule, or combination thereof. The layer formed from this treatment is preferably bound to the activated discrete regions by covalent bonds, electrostatic interactions, or combination thereof.

In one specific embodiment, this invention is directed to a method of further coating the porous materials to provide a double-layer coated porous materials. The second layer is bound to the first layer by covalent bonds, electrostatic interactions, or combination thereof. There is no limit as to the number of coating cycles and thus the number of coating layers that can be introduced on the discrete regions.

In certain embodiments, charged molecules containing the desired functional groups can be reacted with oppositely charged discrete regions to introduce functional groups. Examples of such charged molecules include, but are not limited to, charged polyethylene glycol (PEG), charged biotin, charged DNA, charged RNA, or charged proteins.

### 4. BRIEF DESCRIPTION OF FIGURES

To better understand specific novel aspects of this invention, reference can be made to the figures described below:
Figure 1 provides an illustration of direct sintering of polymer powders to form discrete regions of distinct properties;
Figure 2 illustrates skiving of a porous material following direct sintering to form a sheet of porous material having discrete surface regions;
Figure 3 provides an illustration of sintering a porous material on top of another material to form discrete surface regions;
Figure 4 provides an illustration of selective plasma and corona discharge activation of porous materials using a mask;
Figure 5 illustrates selective corona discharge activation of porous materials without a mask;
Figure 6 illustrates a general schematic of a multi-layer coating process of porous materials;
Figure 7 provides an illustration of a porous material containing functional groups in discrete regions;
Figure 8 provides an illustration of a porous material containing different electric charges in discrete regions;
Figure 9 provides an illustration of three-dimensional discrete porous materials;
Figure 10 illustrates an application of the porous material of this invention in a lateral flow device;
Figure 11 illustrates an application of the porous material of this invention in a 96-well plate;
Figure 12 illustrates an application of the porous material of this invention in a liquid/chemical delivery and microfluidic device; and
Figure 13 illustrates an application of the porous material of this invention in a multi-channel lateral flow diagnostic device.

### 5. DETAILED DESCRIPTION OF THE INVENTION

This invention is directed, in part, to a single piece of porous polymeric material comprising a plurality of discrete surface regions that exhibit distinct surface properties. As used herein, the term "single piece" means that components of the piece are physically connected without third component materials such as glue, adhesive, or tape, etc. The shape of this single piece is not limited to a specific form. Examples of feasible shapes include, but are not limited to, sheets, rods, films, blocks, fibers, tubes, and molded parts.

The distinct properties that can be introduced onto the surface of the porous materials of this invention include, but are not limited to, hydrophilicity, hydrophobicity, oleophobicity, biological molecules binding capability, wetting or wicking property, presence or density of functional groups, chemical reactivity, electric charges, porosity, and pore sizes. A specific embodiment of this invention is directed to a porous material that has discrete hydrophilic and hydrophobic regions. The hydrophilic and hydrophobic discrete porous regions are distinguished by the wetting of aqueous solution at atmospheric pressure. The aqueous solution will wet or wick the hydrophilic areas, but not the hydrophobic areas.

Another specific embodiment is directed to a porous material that has discrete hydrophobic and oleophobic regions. Oleophobicity can be defined by the wetting of organic solvents, such as ethanol, acetone, gasoline, etc., at atmospheric pressure. The organic solvents will not wet or wick the oleophobic areas, whereas such solvents will wet or wick the hydrophobic areas. More specific embodiment is directed to a porous material that has discrete hydrophilic regions surrounded by hydrophobic boundaries.

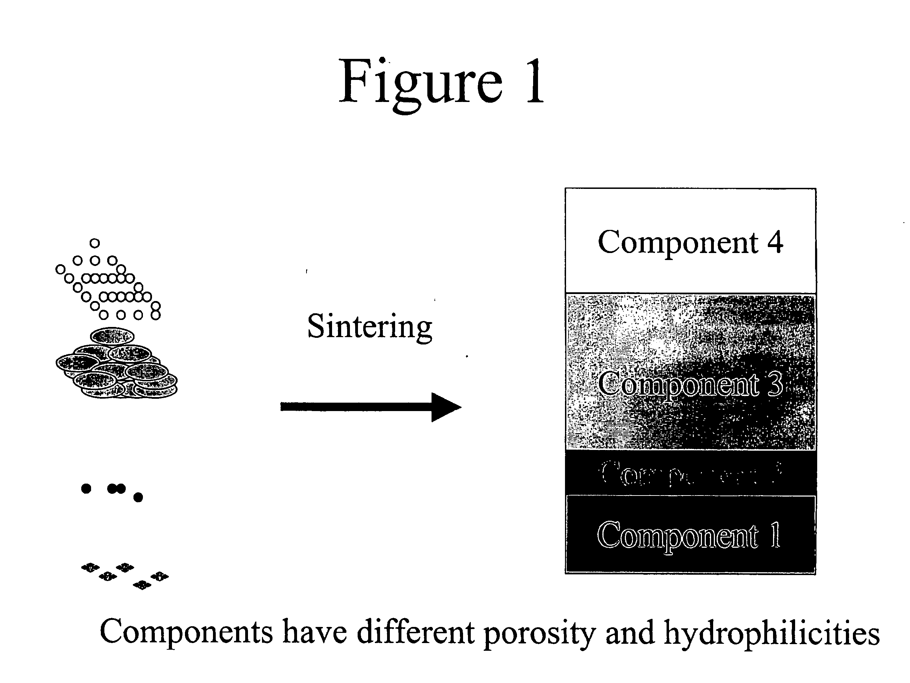

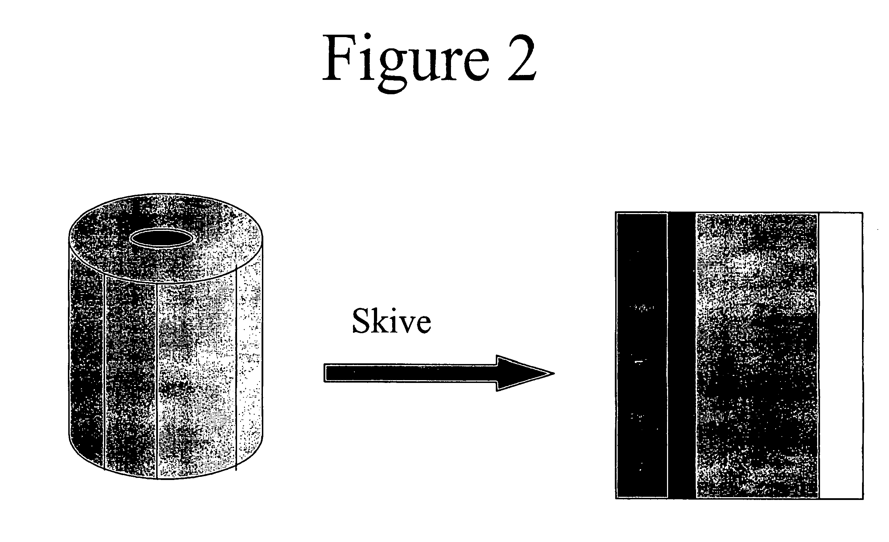

This invention is also directed, in part, to methods of making the porous polymeric material. In one embodiment, the porous materials of the present invention is made by using a method comprising arranging polymer particles that have different properties in a predetermined pattern and compress sintering the particles to provide a porous material (Figure 1). If the resulting porous materials take the form of a billet, they can be optionally skived into sheets (Figure 2).

The porous materials of this invention can also be made by a method comprising providing a porous substrate, selectively placing polymer powders in a predetermined pattern on discrete regions of the substrate, and compress sintering the polymer powders into the substrate. Depending on the types of polymer powders used, same property can be introduced in all discrete regions, or different properties may be introduced in different discrete regions.

In another embodiment, the porous materials of this invention can be made using a method that comprises providing a porous substrate, and selectively activating discrete regions of the substrate. A high-energy activation method is employed for this activation.

Various coating techniques may be employed to provide the porous materials of the present invention. In general, the porous materials are treated with coating materials for a time and at a temperature sufficient for a formation of a layer covering the discrete regions. This treatment can be repeated as desired to achieve a multi-layer coating on the discrete regions of the porous material. Detailed discussion of various aspects of this invention is provided below.

### 5.1 MATERIALS

Materials of this invention can be made using methods described herein from materials such as, but not limited to, those discussed below.

### 5.1.1 Substrates

Substrates that can be used to provide materials of the invention can be solid or porous, and can come in any of a variety of shapes and forms. For example, substrates can be blocks, rods, films, molded parts, tubes, fibers, and sheets.

The solid and porous substrates can be made of a variety of materials, such as, but not limited to: metals (*e*.*g*., Cu, Ag, Au, Al, Zn, and Fe); alloys; glasses; ceramics; carbon black; silica; silicon; and polymeric materials or plastics. As used herein, "porous materials" or "porous substrate" refers to a material or a substrate that has a surface with one or more pores or a surface that is uneven, undulating, or not smooth or flat, such as a woven, non-woven, compressed, perforated, or etched material or substrate.

A specific substrate of the present invention is a porous polymeric or plastic material. Porous polymeric materials can usually be made from a variety of thermoplastic and thermoset materials using methods known in the art such as, but not limited to, sintering and casting. Thus, for example, suitable polymers for the substrate are those that can be sintered to form sheet or membrane-like porous materials. Examples of suitable thermoplastic or thermoset materials include, but are not limited to, polyolefin, polyurethane, polycarbonate, polyether ether ketone (PEEK), poly(phenylene oxide), poly(ether sulfone), polysulfone, nitrocellulose, cellulose, fluorinated polymers, PTFE, porous fiber materials or nylon.

One of the specific substrates are polyolefins. Examples of polyolefins suitable for the present invention include, but are not limited to: ethylene vinyl acetate (EVA); ethylene methyl acrylate (EMA); polyethylenes such as, but not limited to, low density polyethylene (LDPE), linear low density polyethylene (LLDPE), high density polyethylene (HDPE), and ultra-high molecular weight polyethylene (UHMWPE); polypropylenes; ethylene-propylene rubbers; ethylene-propylene-diene rubbers; polystyrene; poly(1-butene); poly(2-butene); poly(1-pentene); poly(2-pentene); poly(3-methyl-1-pentene); poly(4-methyl-1-pentene); 1,2-poly-1,3-butadiene; 1,4-poly-1,3-butadiene; polyisoprene; polychloroprene; poly(vinyl acetate); poly(vinylidene chloride); poly(vinylidene fluoride); poly(tetra fluoro ethylene); and mixtures and derivatives thereof.

Specific polyethylene materials include, but are not limited to, those in the Exact^{®} series manufactured by Exxon Chemical Company, such as Exact SLX-9090, Exact 3024, Exact, 3030, Exact 3033, Exact 4011, Exact 4041, Exact SLP-9053, Exact SLP-9072, and Exact SLP-9095. Specific examples of LDPE include, but are not limited to, those in the 20 series manufactured by DuPont Chemical Company, Wilmington, Delaware, such as 20 series 20, 20 series 20-6064, 20 series 2005, 20 series 2010, and 20 series 2020T. Specific examples of LLDPE include, but are not limited to, those in the Exact^{®} series manufactured by Exxon Chemical Company, such as Exact 3022 and Exact 4006. Specific examples of HDPE include, but are not limited to, those in the Escorene HX^{®} series manufactured by Exxon Chemical Company, such as Escorene HX-0358.

Ultra-high molecular weight polyethylenes include, but are not limited to, UHMWPE having a molecular weight greater than about 1,000,000. Typically, UHMWPE displays no measurable flow rate under normal test procedures. *See*, U.S. patent no. 3,954,927, herein incorporated by reference. Ultra-high molecular weight polyethylene also tends to have enhanced mechanical properties compared to other polyethylenes, including, but not limited to, abrasion resistance, impact resistance and toughness. Polyethylenes having weight average molecular weights of 1,000,000 or higher, which are included within the class designated as UHMWPE, typically an intrinsic viscosity in the range of about 8 or more. Specific examples of UHMWPE include, but are not limited to, Hostalen GUR^{®} sold by Ticona Inc., League City, Texas.

Polypropylenes include, but are not limited to: the Polyfort^{®} series manufactured by A Shulman Co., Akron, Ohio, such as FPP 2320E, 2321E, 2322E, 2345E, PP2130, and PP2258; the Acctuf^{®} series manufactured by BP Amoco Corporation, Atlanta, Georgia, such as Acctuf 3045, Amoco 6014, and Amoco 6015; the Aristech^{®} series manufactured by Aristech Chemical Corp., Pittsburgh, PA, such as D-007-2, LP-230-S, and TI-4007-A; the Borealis^{®} series manufactured by BASF Thermoplastic Materials, Saint Paul, Minnesota, such as BA101E, BA110E, BA122B, BA204E, BA202E, and BA124B; the Polypro^{®} series manufactured by Chisso America Inc., Schaumburg, Illinois, such as F1177 and F3020; the Noblen^{®} series manufactured by Mitsubishi Petrochemical Co. Ltd., Tokyo, Japan, such as MA8; the Astryn^{®} series manufactured by Montell USA Inc., Wilmington, Delaware, such as 68F4-4 and PD451; the Moplen^{®} series manufactured by Montell USA Inc., such as D 50S, D 60P, and D 78PJ; and the Pro-Fax^{®} series manufactured by Montell USA Inc., such as 6723, 6823, and 6824.

As a practical matter, the term "pore" is an artificial one that can have various meanings. According to the present invention, the average sizes, shapes, and number of pores in a material can be determined by taking a cross-section of the material. For the purpose of this invention, holes and depressions in the cross-section are considered pores. And, while only two-dimensional sizes and shapes of those pores can be determined from the cross-section, information about their third dimension (*e*.*g*., their depth) can be determined from a second cross-section, orthogonal to the first. Also, average pore size, pore volume, and/or surface area can be inferred from measurements obtained using a mercury intrusion porisometer. For the purpose of this invention, pore sizes are typically referred to in terms of their average diameters, even though the pores themselves are not necessarily spherical.

The particular method used to form the pores or channels of a porous polymeric material and the resulting porosity (*i*.*e*., average pore size and pore density) of the porous material can vary according to the desired application for which the final membrane be used. The desired porosity of the matrix can also be affected by the polymeric material itself, as porosity can affect in different ways the physical properties (*e*.*g*., tensile strength and durability) of different materials. For the purpose of this invention, pore size and pore density can be determined using, for example, a mercury porisometer and scanning electron microscopy. Specific porous substrates of this invention have an average pore size of from about 0.001 µm to about 1000 µm, more specifically from about 0.01 µm to about 500 µm, from about 0.1 µm to about 200 µm, more specifically from about 1 µm to about 100 µm, more specifically from about 1 µm to about 50 µm, from about 1 µm to about 20 µm, even more specifically from about 1 µm to about 10 µm.

Although the porous polymeric material of the present invention can be made from the materials discussed above, many other materials that are commercially available can also be used for the purposes. Suitable substrates can be purchased from Porex Technologies, Fairburn, GA.

### 5.1.2 Coating Materials

In one embodiment of this invention, the discrete regions of the porous materials are further coated using treatments by coating materials. Coating of discrete porous materials can be single layered or multilayered, preferably multilayered. The multilayer coating of the invention comprises at least two layers, the first of which is adhered (*e*.*g*., covalently or electrostatically) to the surface of discrete hydrophilic regions, and the second of which is adhered (*e*.*g*., electrostatically or covalently) to the first layer. Using the methods disclosed herein as well as the ones known in the art, additional layers can be adhered atop the second layer and to one another. Suitable coating materials include, but are not limited to, polyelectrolytes, neutral polymers, small molecules, biomolecules, or combinations thereof.

Certain materials forming coating layer contain net cationic or anion charges, or localized cationic or anionic charges (*e*.*g*., zwitterions). Alternatively, they can provide net or localized charges when adhered or deposited onto the discrete porous materials. Other materials forming coating layer may contain functional groups that can form covalent bonds with the discrete porous materials under certain conditions (*e*.*g*., dry, heat, coupling reagents).

As used herein, unless otherwise indicated, the term "polyelectrolyte" means a polymer having electric charges. The polyelectrolyte may exist in a complex form, which is also called symplexes. Polyelectrolytes are divided into polyacids, polybases, and polyampholytes. Depending on the charge density in the chain, polyelectrolytes are divided into weak and strong. The charge of weak polyelectrolytes is determined by dissociation constants of ionic groups and pH of the solution. Strong polyelectrolytes in water solutions are mostly ionized independent of the solution's pH. Typical weak polyacid polyelectrolytes include, but are not limited to, poly(acrylic acid) and poly(methacrylic acid). Strong polyacid polyelectrolytes include, but are not limited to, poly(ethylenesulfonic acid), poly(styrenesulfoinic acid), and poly(phosphoric acid). Weak polybase polyelectrolytes include, but are not limited to, poly(4-vinylpyridine), polyethyleneimine (PEI), and polyvinylamine. Strong polybase polyelectrolytes can be obtained by alkylation of nitrogen, sulfur, or phosphorus atoms of weak polybase polyelectrolytes. See "Concise Polymeric Materials Encyclopedia" (Joseph C. Salamone, 1999 by CRC Press LLC, ISBN 0-84932-226-X, pages 1140 - 1141). Other examples of polyelectrolytes include, but are not limited to, alginic acid, adipic acid, chemical dyes, proteins, enzymes, nucleic acids, peptides, or salts, esters, and/or copolymers thereof.

Organic materials that can be used to form coating layer on discrete hydrophilic areas of the invention include, but are not limited to, organic polymers, monomers, and biomolecules. Organic materials may contain net and/or localized cationic or anionic charges. Organic materials that are preferred for direct adhesion to the discrete porous materials are polymers such as, but not limited to, single and copolymers (*e*.*g*., random, graft, and block copolymers).

Examples of polymers or copolymers that contain cationic charges include, but are not limited to, polyethyleneimide (PEI), poly(vinylimidazoline), quaterized polyacrylamide, polyvinylpyridine, poly(n-vinylpyrrolidone), polyvinylamines, polyallylamines, chitosan, polylysine, poly(acrylate trialkyl ammonia salt ester), cellulose or other polymers that contains quaternizied groups of nitrogen, phosphor and polymer surfactants, or salts, esters, and/or copolymers thereof.

Examples of polymers or copolymers that contain anionic charges include, but are not limited to, polyacrylic acid (PAA), polymethylacralic acid, poly(styrenesulfuric acid) (PSSA), and their sodium salts, cellulose that contain sulfonated or carboxylic acid groups, carboxyl modified polyacrylamide, poly(vinylsulfonic acid), poly(toluene sulfuric acid), poly(methyl vinyl ether-alt-maleic acid) and ester, poly(glutamic acid), poly(maleic acid), Nafion^{®} (DuPont), dextran sulfate, hyaluric acid, heparin, sodium carboxymethyl cellulose (CMC), anionic charged polymer surfactants, molecules containing phosphate groups, or salts, esters, and/or copolymers thereof.

Coating of hydrophilic areas on this invention can also be made from biomolecules. Certain biomolecules contain net or localized charges. Examples of biomolecules include, but are not limited to, biotin, proteins, enzymes, lipids, hormones, peptides, nucleic acids, oligonucleic acids, DNA, RNA, sugar and polysaccharides.

Polymers and copolymers that contain both cationic and anionic moieties can also be used to provide coating on the hydrophilic areas of this invention. For example, about one to about 99 percent of repeat units of a polymer can contain cationic moieties, preferably from about 20 to 80 percent. Amphoteric polymers (*i*.*e*., polymers wherein about 50 percent of the repeat units contain cationic groups and about 50 percent contain anionic groups) can also be used. Polymers and copolymers may have varying charge density (i.e., ratio of number of charge to the number of repeat units). For example, polymers with charge densities of from one to 100 percent, preferably from about 20 to about 100 percent, can be used.

Neutral polymers can also be used to form the multi-layers coating on hydrophilic areas of the invention, particularly polymers capable of forming covalent bonds with the components of other layers or with substrate surface under conditions such as those discussed herein. Examples of such neutral polymers include, but are not limited to: isocyanated terminated polymers, including polyurethane and PEG; epoxy-terminated polymers, including PEG and polysiloxanes; and hydroxylsuccimide terminated polymers.

The polymers and copolymers of the invention can be linear, comber, star or cyclic polymers. The copolymers can be random, graft, or block copolymers.

Molecules other than traditional polymers (*e*.*g*., small organic and inorganic molecules) can also be used to provide layers and coatings on the hydrophilic areas of the invention. These molecules can be used in any layer in the multi-layer coating, preferably, in the secondary or sequential layers, and more preferably be used combined with the polymer layers. The molecular weight of these molecules varies from 50 to a million, preferably from 50 to 10,000. Preferably these molecules are electrically charged. Examples of these molecules include, but are not limited to, surfactants, phosphates, bromates, sulfonates, tertiary amines, dyes, lipids and metal ions. Examples of the surfactants of the invention include, but are not limited to, Zonyl Surfactants (DuPont), SUPFYNOI surfactant (Air product), FLUORAD (3M), sodium dodecylsulfonate (SDS), Dodecyltrimethylamonium bromide (DTAB).

Metal ions can also be used to form the coating on the hydrophilic areas of the inventions, in certain cases with more than one positive charges, and can form strong complex with organic functional groups such as, but not limited to, amino, carboxylic acid, thiol, sulfide, or disulfide groups.

### 5.2 MANUFACTURE OF DISCRETE POROUS MATERIALS

Discrete porous materials of this invention can be prepared using the methods described herein.

### 5.2.1 Direct Sintering of Polymer Powders

Sintering is a process that fuses discrete particles and/or substrates, such as polymer particles and substrates, together by heat. For example, polymer particles can be first packed in a mold or other containers or substrates. The particles are then heated to a temperature that usually melts only the outer surface or shell of the particles. The particles are then fused together at this temperature and cooled down to a lower temperature, such as room temperature, to form the sintered product. Many suitable sintering process of making a porous polymer can be used to form the sintered porous polymeric material of the present invention.

In one embodiment, polymer powders with different compositions are selectively laid on specific areas of a compression mold. The laid down polymer powders then are compress sintered into finished parts (Figure 1). In the finished parts, some areas are hydrophilic and other regions are hydrophobic. The geography of the discrete hydrophilic and hydrophobic regions is determined by the powder position before the sintering. The resulting porous material may take the form of a billet, in which case the porous material may be optionally skived into sheets (Figure 2).

Discrete hydrophilic and hydrophobic porous material can also be manufactured by selectively placing different powders on different locations of a mold. These different powders will provide different hydrophilicity after the sintering. After the sintering, the polymer powders would fuse into the substrate and form self-sustained porous plastic parts. These finished hydrophilic and hydrophobic discrete parts, if in the form of a billet, can be used as is or may further be skived into sheets or films. (Figure 2).

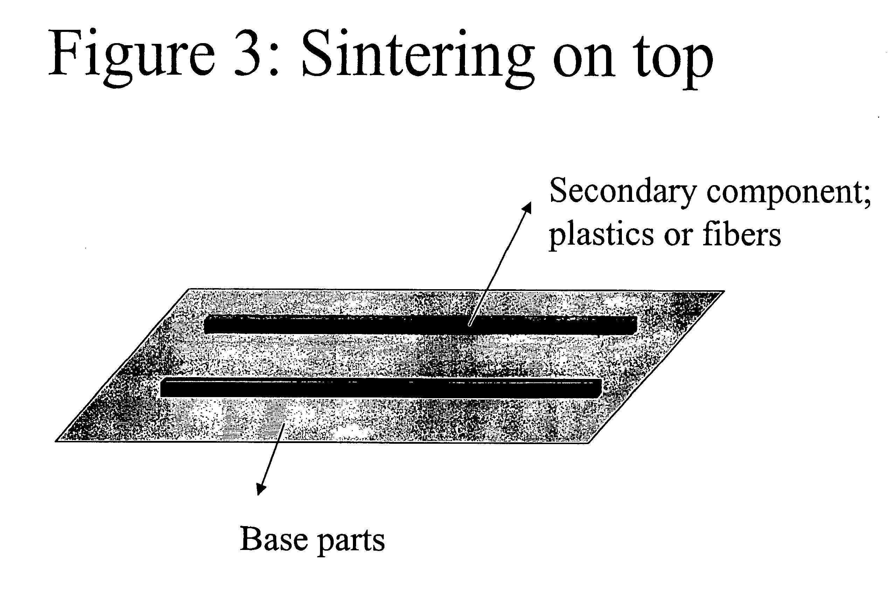

Discrete hydrophilic and hydrophobic porous material can also be manufactured by selectively placing hydrophilic powders on top of hydrophobic porous materials or other solid non-porous materials and sintering them under an elevated temperature. After the sintering, the hydrophilic polymer powders were fused together and form a single piece with the substrate materials. (Figure 3).

After one or more of the sintering processes discussed herein, the discrete hydrophilic and hydrophobic regions may either have the same porosity and pore sizes or different porosity and pore sizes. Different porosity and pore sizes can be obtained on discrete areas of porous material by applying powders with different shapes and sizes to different discrete areas. Different porosity and pore sizes can also be achieved by using the same powder materials but applying different pressures on different discrete regions of the porous materials during or after the sintering process, or by adjusting the duration of sintering time.

Each discrete hydrophilic area can have the same kinds of porous materials and same wicking property. Each discrete hydrophilic area may have the same kind of porous material, but with different porosities, pore sizes and wicking properties. Further, each discrete hydrophilic area may also have different porous materials. For example, certain discrete areas may be made of polyethylene, while other discrete areas may be made of PTFE material. There are many combinations for the sinterable porous materials that can be used to manufacture discrete porous materials.

Those skilled in the art will recognize that the average pore size of the porous polymeric material will depend, at least in part, on the average particle size of the polymeric material, the sintering temperature, and the pressure, if any, applied to the mixture during sintering. For example, in most cases, if the particles of the other optional materials are smaller than the average pore size of the porous material, they will be trapped within pores of the material during the sintering process, and may be adhered to the walls of those pores. On the other hand, if particles of the other optional materials are larger than the average pore size of the porous material, they will be incorporated within the porous material as inclusions. In the case that the melting point of optional material is lower than that of the porous material, the optional material may coat the pores.

Sintering can occur on a solid support or within a mold to yield a final product that can be cut into pieces of desired shape. The use of molds is preferred where the desired shape of the self sealing medium is complex.

### 5.2.2 Surface Activation

According to the present invention, the surface of a substrate can be activated using one or more methods known in the art such as, but not limited to, chemical treatment, plasma discharge, electron-beam discharge, corona discharge, and UV radiation. This activation alters the surface of the substrate (*e*.*g*., cleaving chemical bonds) to allow the formation of hydrophilic and/or chemically active moieties such as, but not limited to, hydroxy, amine, and carboxylic groups. As one of ordinary skill in the art understands, the particular functional groups formed will depend on the chemical composition of the substrate surface and the methods and conditions used to activate it. Often, the activation of a hydrophobic plastic surface usually provides a hydrophilic, electrically charged surface. High energy activation methods such as direct and remote plasma activation, corona discharge, electron beam, and UV radiation are preferred in this invention.

Plasmas that can be used to provide negatively charged porous plastic surfaces include, but are not limited to, plasmas of argon, oxygen, nitrogen, methanol, ethylene oxide, and acetone. Plasmas that can be used to provide positively charged surfaces include, but are not limited to, ammonia and ethylenediamine. Depending on the composition of the substrate, its size, and the particular plasma used, the time necessary to achieve a desired surface will vary. Typical times can vary from about 1 minute to about an hour. Similarly, the power necessary to achieve the desired plasma may vary from about 50 W to about 1000 W.

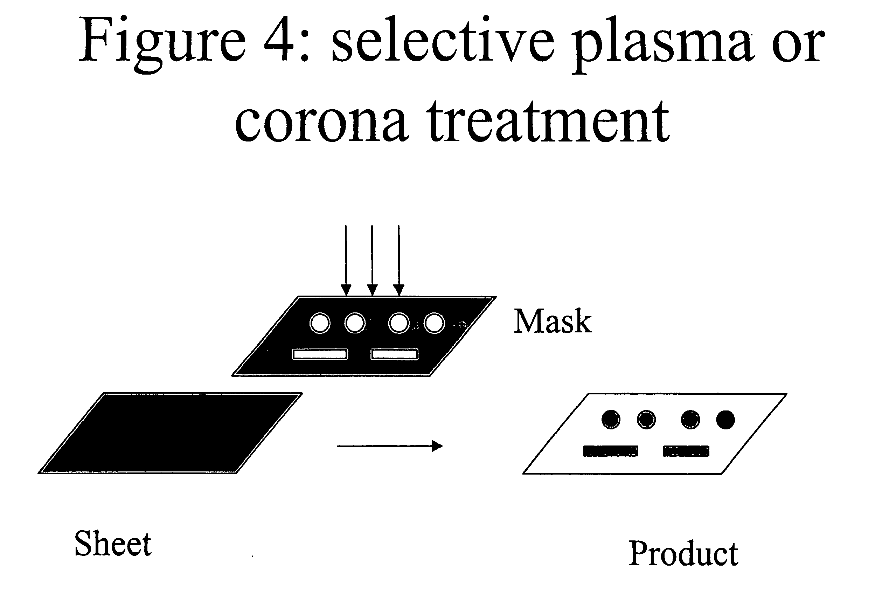

Creating discrete hydrophilic and hydrophobic areas on porous materials requires selectively blocking plasma radiation on the hydrophobic areas. After the plasma treatment, only the areas that have been exposed to the plasma discharge become hydrophilic and the areas that are blocked by, for example a mask, will retain their hydrophobic property (Figure 4). The mask materials can be anything that can form tight contact with the porous materials and effectively block the ions and radicals from the plasma. These materials include, but are not limited to, metals, plastics, rubbers, and paper tapes. A specific mask material is polyester.

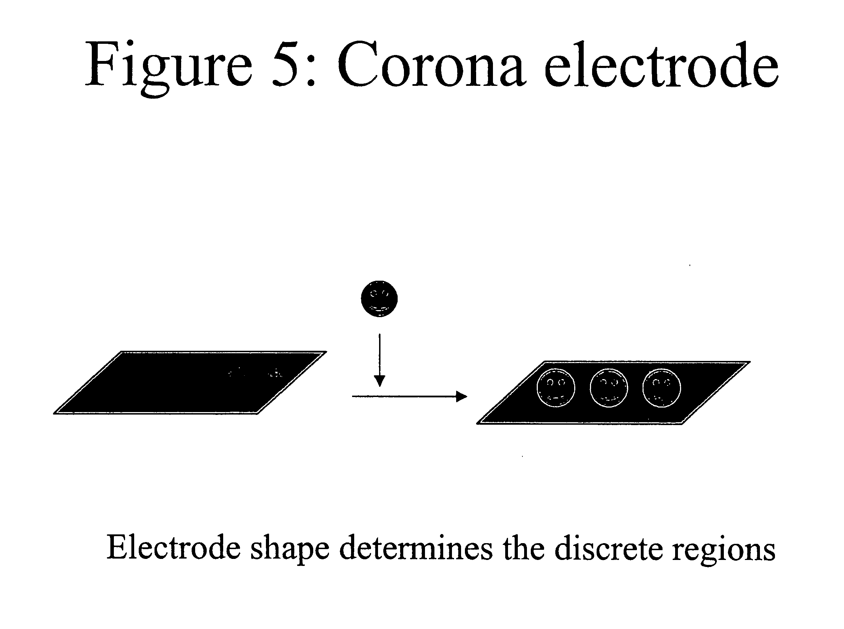

Corona discharges can also be used to manufacture discrete hydrophobic and hydrophilic porous materials. There are two different ways to manufacture discrete hydrophobic and hydrophilic porous materials using corona discharges. One is to apply a mask that contacts tightly with uniform porous materials, so that after the masked porous materials pass through the corona discharge area, only the unmasked parts of porous materials become hydrophilic (Figure 4). The masked parts of porous materials will retain their hydrophobic property. Another way to manufacture discrete hydrophobic and hydrophilic porous material is by using specially designed electrodes. These electrodes are specially made to the shape that is identical to the geography of discrete hydrophobic and hydrophilic regions of the porous material. When the uniform porous materials pass the electrodes, the electrodes will make the porous materials right below them become hydrophilic. The areas not encompassed by the electrodes will still retain their hydrophobic property (Figure 5).

Electron beam is another method that can be used for selectively activating discrete regions on porous materials. For selective activation, electron beam can simply be focused to specific areas of porous materials. The areas to which the electron beam is focused will become hydrophilic after the electron beam treatment. Areas not exposed to the electron beam will retain their original hydrophobicity.

### 5.2.3. Coating of the Activated Discrete Regions

After the uniform porous materials are selectively activated and discrete regions of hydrophilic and hydrophobic characteristics are formed, the discrete hydrophilic areas can be further enhanced or fixed by coating. Any methods of surface modification may be applied for this purpose. For example, treatment of the activated discrete regions by polyelectrolyte solutions may be employed. Likewise, method of coating the porous materials with functional additives, as disclosed in co-pending U.S. patent application no. 09/866,842, herein incorporated by reference, can also be used for certain purposes. Method of coating the porous substrates with various coating materials, as disclosed in co-pending U.S. patent application no. 10/228,944, herein incorporated by reference, can also be used.

### 5.2.3.1 Treatment by Polyelectrolyte Solutions

In a specific embodiment of the present invention, the discrete regions of the porous material of this invention may be subjected to sequential treatment by polyelectrolyte solutions. The substrate is contacted with a solution of the materials from which the first layer will be formed on the surface of the substrate. Specific suitable solutions are solutions of cationic or anionic polymers. The solutions can be aqueous, but organic solvents can also be used. Specific examples are solutions of water, ethanol, isopropanol, and mixtures thereof. The contact between the substrate and the solution is maintained for a sufficient time and at a sufficient temperature for the first layer to form on the substrate surface. Specifically, layers are formed by the formation of covalent bonds and/or electrostatic interactions between functional groups on the substrate surface and molecules in the solution.

The interactions between functional groups on the substrate surface and molecules in the solution can be adjusted by the type of solvent used, temperature, pH and the addition of coupling agents. For example, high pH and coupling agent concentration can promote covalent bond formation between the substrate and the first layer of coating.

After the resulting coated substrate is removed from the solution, it is washed with, for example, deionized water in an ultrasonic bath. Typical wash times will vary depending on the solvent and the materials used to form the first layer, but are often about 10 minutes or less. Optionally, the washed, single-layer coated substrate can be dried at an elevated or room temperature. Elevated temperature promotes formation of covalent bonds.

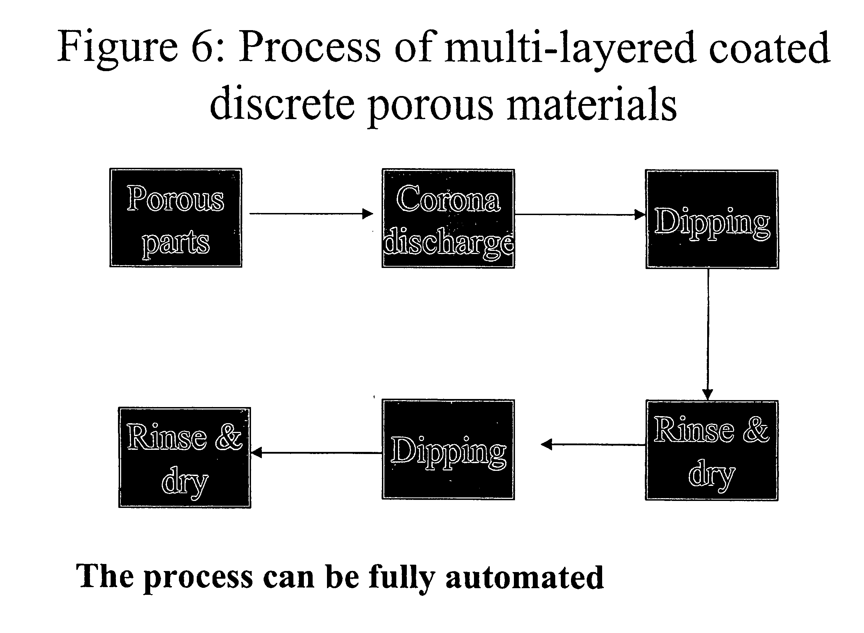

The single-coated substrate can then be contacted with a second solution. Preferably, this second solution is of molecules that are of an opposite charge to those that form the first layer so that the second layer adheres to the first via electrostatic interactions. However, the first layer can also be formed from molecules that have functional groups that, with or without activation, can react with functional groups on the molecules used to form the second layer. After the resulting dual-coated substrate is removed from the second solution, it is preferably washed and dried at room temperature or an elevated temperature. An illustration of the coating process is shown in Figure 6.

Polyelectrolyte solutions having a concentration of about 10 ppm to about 100,000 ppm are typically used for the purpose of the present invention. As those of ordinary skill in the art will appreciate, the concentration of any particular solution depends on the polymer molecular weight, charge density and type of molecules from which a given layer is to be made. Solutions of higher molecular weight molecules generally require lower concentrations than those of lower molecular weight molecules. Similarly, high ionic density polymers typically require lower solution concentrations. For solutions such as PEI and PAA, specific concentrations are in the range of about 0.05 % to about 2 %, or about 0.1 % to about 1 %, in ethanol-water solution. Generally, biomolecules show high immobilization on a surface with opposite electric charges, particularly when the media is of low ionic strength.

Electrostatic interaction is one of the most important interactions between differently charged polyelectrolytes, especially during complex formation. Different polyelectrolytes can also form covalent bonds between their functional groups. For example, the amino group in PEI can form amide bond with the carboxylic acid group in PAA. The formation, strength, and durability of the covalent bonds also depend on the type of solvent, temperature, pH and presence and concentration of coupling agents. The ratio of PEI and PAA and the coupling agent will also have an effect on the percentage of covalent bond formed. Coupling reagents, such as dicyclohexylcarbodiimide (DCC) and 1-ethyl-3-(3-dimethylaminopropyl)carbodiimide (EDC), can be used to promote such reactions.

Examples of different coating scenarios include: electrostatic interactions between substrate and first layer and covalent bond between first layer and second layer; covalent bond between substrate and first layer and covalent bond between first and second layer; electrostatic interactions between substrate and first layer and electrostatic interactions between first and second layer; and mixed covalent bond and electrostatic interactions for both coatings. Because the molecules forming each layer can be bound to the material below it by multiple covalent and/or electrostatic interactions, typical materials of the invention have highly stable coatings that are resistant to delamination and/or dissociation. Furthermore, the high stability of the present invention's multilayer coating results in lower solubility of the coatings, and thus, provides coatings with low leaching.

The process of optionally activating a porous surface and contacting it with a solution of one or more compounds under conditions sufficient to form a layer on the surface can be repeated to achieve coating of multiple layers. Thus, multi-layer coating of varying thickness, density, and uniformity can be adhered to the discrete surfaces of variety of porous materials.

### 5.2.3.2 Porous Materials with Discrete Functional Groups in Different Areas

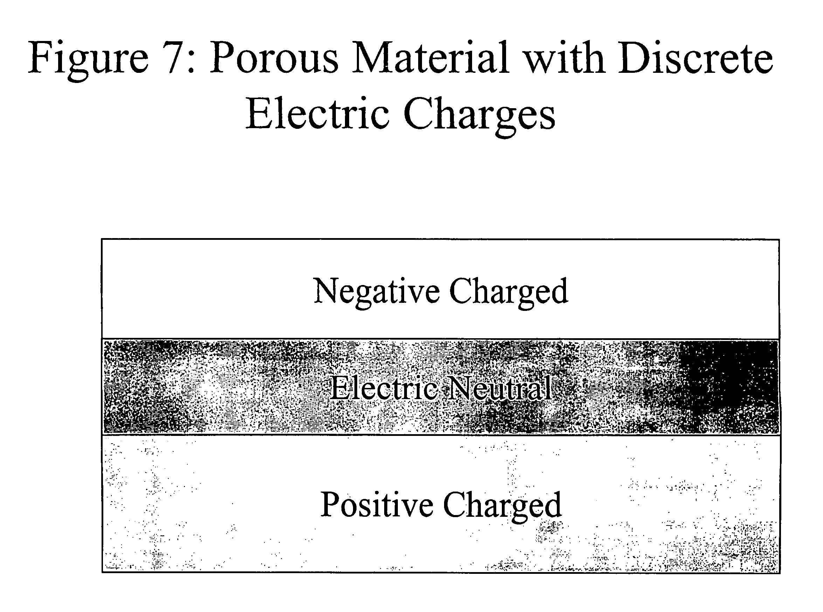

This invention also provides discrete porous materials substrates with a variety of chemically reactive functional groups (Figure 7). The following variations can be achieved using the method of the present invention: each discrete hydrophilic area has the same kinds of reactive functional groups; each discrete hydrophilic area has the same chemically reactive functional groups, but different functional group density; and further, each discrete hydrophilic area may also have different chemically reactive functional groups.

By way of an example, functional groups that can be introduced onto discrete porous materials areas (*e*.*g*., porous plastics) include amino groups (including primary, secondary and tertiary amines), which are typically positively charged at neutral pH. Amino-functional discrete porous materials can be manufactured by any of the following methods: selectively activating porous material in amine containing (*e*.*g*., ammonia, ethyldiamine) plasma chamber or corona discharge environment; coating PEI or other amino group containing polyelectrolytes solutions on discrete activated hydrophilic area; or coating PEI or other amino group containing polyelectrolytes solutions to the discrete porous materials having negatively charged coating on them.

Carboxylic acid groups can be introduced onto porous materials by treating positively charged porous materials with PAA or other carboxylic acid containing polyelectrolytes solutions. Typically, positively charged materials have either been treated with a positively charged polyelectrolyte or have been activated in ammonia solution or ammonia plasma.

Sulfuric acid functional groups can be introduced onto porous materials by treating positively charged porous materials with poly(styrenesulfuric acid) (PSSA) or other sulfuric acid containing polyelectrolytes solutions. Typically, positively charged materials have either been treated with a positively charged polyelectrolyte or have been activated in ammonia solution or ammonia plasma.

PEG molecules can be coated onto charged porous materials by treating charged porous materials with PEG molecules that contain functional groups with opposite charges. For example, a PEG molecule having a carboxylic acid functional group can be coated onto a porous material coated with PEI.

Biomolecules can also be coated onto the substrates of this invention. For example, biotin, which is a small biomolecule that can specifically bind to avidin and streptavidin, can be introduced onto porous materials by treating charged porous materials with the biotin derivatives that contain opposite charges as compared to the porous materials.

Many polysaccharides contain electric charges and can provide good matrices for cell growth and harvesting. These charged polysaccharides, such as heparin, chitosan, and CMC, can be introduced onto porous materials by treating oppositely charged porous materials with the polysaccharides.

Fluoroalkyl groups, such as perfluoroalkyl groups, can be attached to porous materials by treating charged porous materials with fluoroalkyl molecules that contain opposite charges.

### 5.2.3.3 Porous Materials with Discrete Electric Charges in Different Areas

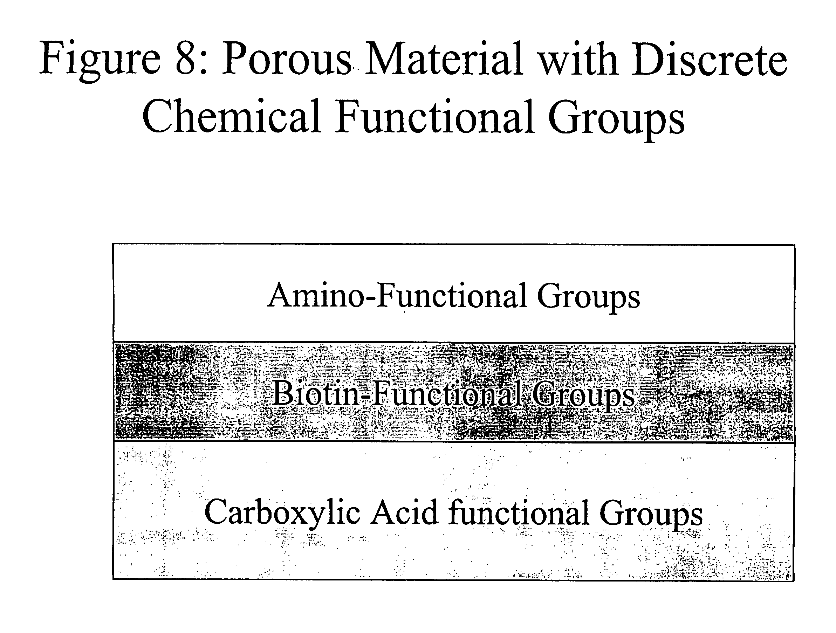

Methods of this invention can provide discrete porous materials substrates with a variety of electric charges and charge density (Figure 8). Variations that can be achieved using the methods of the present invention include: porous materials wherein each discrete hydrophilic area has the same kind of electric charges; porous materials wherein each discrete hydrophilic area has the same electric charges, but different charge density; and porous materials wherein different discrete hydrophilic areas have different electric charges.

For example, positive charges can be introduced onto discrete porous material areas (*e*.*g*., porous plastics) by applying PEI solution treatment on specific areas. For the purpose of this invention, the concentration of PEI solution ranging from about 0.05 % to about 1 % is optimal. PEI coated porous materials are typically positively charged at neutral pH. Negative charges can be introduced onto discrete porous material areas (*e*.*g*., porous plastics) by applying PAA solution treatment on specific area. For the purpose of this invention, the concentration of PAA solution ranging from about 0.05 % to about 1 % is optimal. PAA coated porous materials are typically negatively charged at neutral pH. Neutral electric charges can be introduce to porous material by applying neutral or amphoteric (*e*.*g*., peptides) polymers to make porous materials with neutral electric charge at neutral pH.

### 5.2.4 Three-dimensional Porous Materials

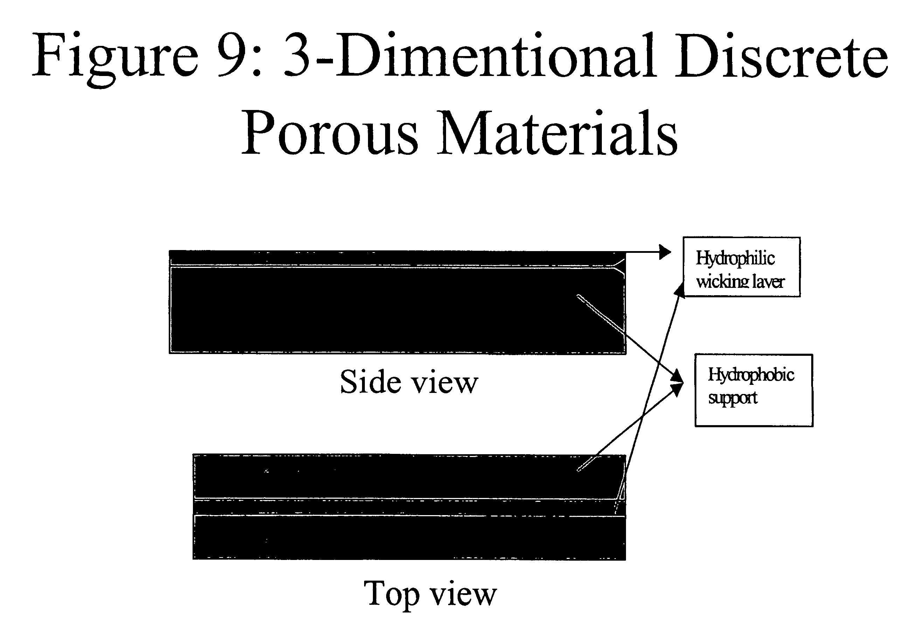

Discrete porous parts in the invention can also be in the z-direction, or combination of x-y-z direction (Figure 9). Certain parts of porous material depth would have different hydrophilicity, porosity, pore sizes, functional groups and electric charges than other parts of the depth. Any methods of making discrete porous materials disclosed herein can be applied to manufacture three-dimensional porous materials. In making three-dimensional discrete porous materials, the activation energy of plasma, corona and electron beam must be controlled to limit the penetration depth of discharges. The conditions for controlling the activation energy vary with the materials and can be readily determined by the persons of ordinary skill in the art.

### 5.2.5 Applications of Discrete Porous Materials

Materials of the present invention have a wide variety of applications. Without limitations, the applications of the discrete porous materials of the present invention include: liquid and chemical delivery; microfluidic devices; lateral flow devices; flow through devices; filtration devices; solid state synthesis devices; extraction devices; cell growth devices; and implant materials. The areas of the application include, but are not limited to: immunoassay; diagnostics; medical devices; separation and purification; fast screening; combinatory chemistry; pregnancy, HIV, and drug abuse tests; and cell biology.

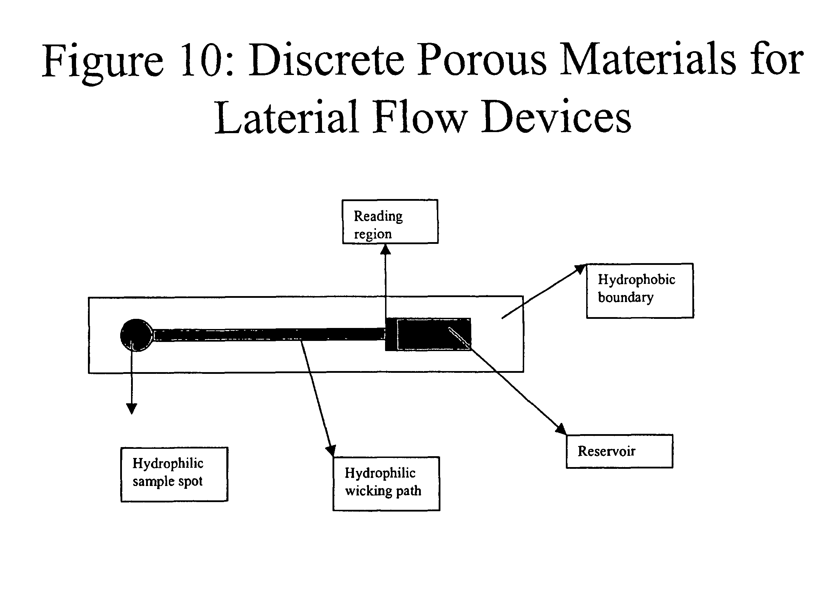

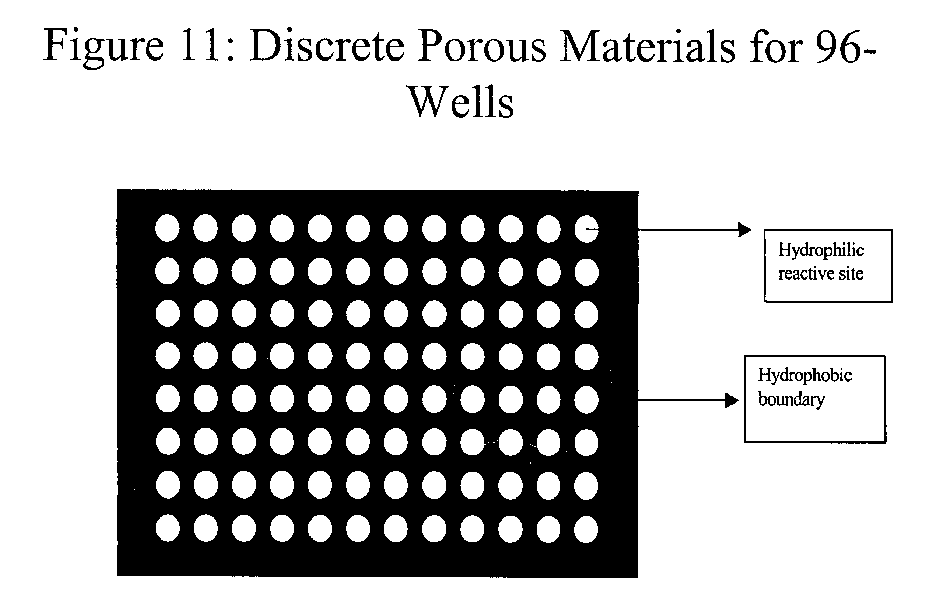

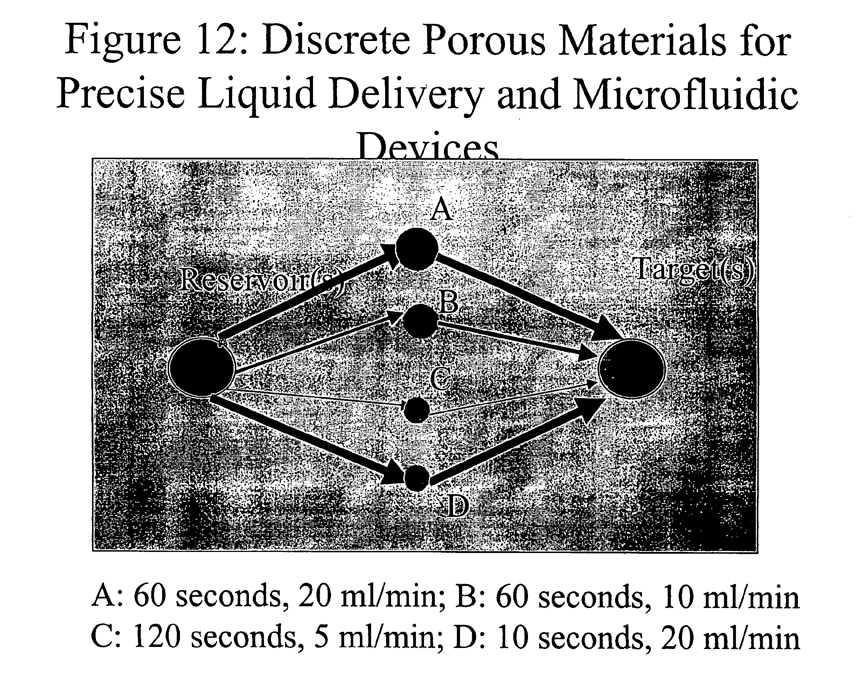

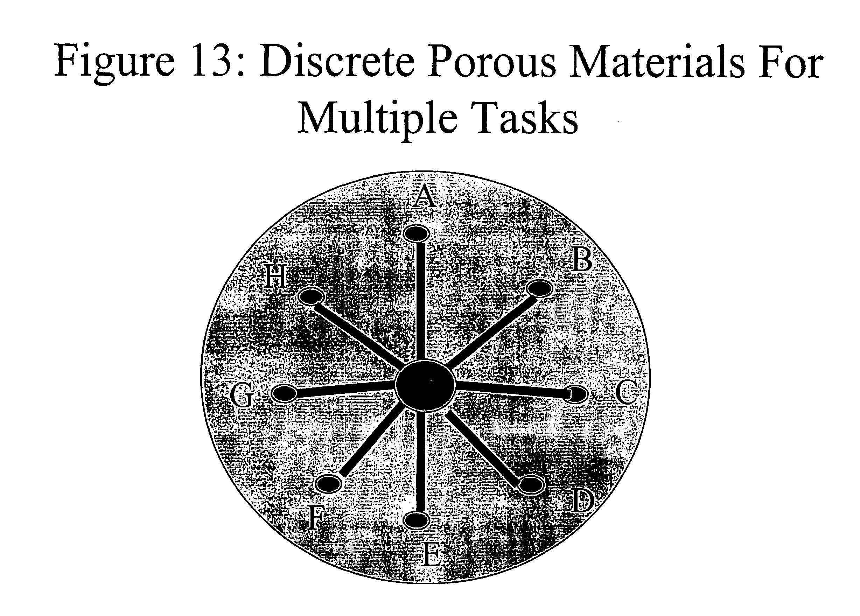

Illustrations of some of the applications are shown in figures as follows: Figure 10 illustrates an application of discrete porous materials of this invention in a single piece lateral flow diagnostic device; Figure 11 shows an application of discrete porous materials of this invention in discrete reactive zones containing 96 wells; Figure 12 shows an application of discrete porous materials of this invention in liquid and chemical delivery systems or microfluidic devices, wherein the liquids can be delivered from beginning spot to the destination through different routes with different time, rates and chemicals; and Figure 13 illustrates an application of discrete porous materials of this invention in multi-channel lateral flow devices. These figures are provided for illustration purpose only. It will be readily apparent to the person of ordinary skill in the art that numerous variations can be contemplated and/or practiced without departing from the scope and spirit of the present invention.

### 6. EXAMPLES

Certain embodiments of this invention, as well as certain advantages of this invention, are illustrated by the following non-limiting examples. Although limited number of examples are disclosed herein, it will be apparent to those skilled in the art that many modifications, both to materials and methods, may be practiced without departing from the purpose and interest of this invention.

### 6.1 ONE STEP SINTERING DISCRETE HYDROPHILIC-HYDROPHOBIC POROUS SHEET MATERIALS

Polymer powder A (UHMW, polyethylene) and Powder mixture B (UHMW, polyethylene, wicking reagent) were laid down to a compression mold at a stripes with width of 1 cm alternatively. The laid down polymer powders were sintered at an elevated temperature and single porous plastic sheet was resulted. In this single plastic porous sheet, the area of polymer powder A was hydrophobic and the area of polymer powder B was hydrophilic (Figure 1).

### 6.2 SINTERING DISCRETE HYDROPHILIC-HYDROPHOBIC POROUS BILLET MATERIALS AND SKIVING INTO DISCRETE SHEET MATERIALS

Polymer powder A (UHMW, polyethylene) and Powder mixture B (UHMW, polyethylene, wicking reagent) were selectively loaded to a 12 inch high and 5 inch diameter round column shape mold. The column was divided equally to 8 sections along the axis with a solid polymer sheet (0.2 cm thick). The powder A and powder B were loaded to the column mold alternatively. The solid polymer strips can be either removed from or remain inside the mold. The polymer powders inside the mold were then sintered at an elevated temperature and single porous plastic billet was resulted. The billet was then skived into thin sheet. The resulting sheet had discrete hydrophilic-hydrophobic areas.

The area of polymer powder A became hydrophobic and the area of polymer powder B became hydrophilic (Figure 2). If solid non-porous boundary was not removed, the resulting materials could also contain discrete porous and non-porous regions.

### 6.3 ONE STEP SINTERING MOLDED PARTS WITH DISCRETE HYDROPHILIC AND HYDROPHOBIC AREAS

Polymer powder A (UHMW, polyethylene, 60 micron) and Powder mixture B (UHMW, polyethylene, 120 micron, wicking reagent) were selectively loaded to a 1 inch high and 0.5 inch diameter round column shape mold. The column was packed with 0.8 inch of powder B at the bottom and 0.2 inch of powder A on the top. The polymer powders inside the mold were then sintered at an elevated temperature. The resulting part had discrete hydrophilic-hydrophobic areas. The area of polymer powder A on the top 0.2 inch became hydrophobic and had a smaller pore size. The area of polymer powder B at the bottom 0.8 inch became hydrophilic and had a larger pore size.

### 6.4 MANUFACTURE OF DISCRETE HYDROPHILIC-HYDROPHOBIC POROUS MATERIALS BY SELECTIVE PLASMA TREATMENT

A sintered porous plastic sheet (T3, Porex Corporation, 0.4mm thick) was tightly covered with a polyester tape mask on both sides of the plastic sheet. The covered porous plastic sheet was then exposed to a treatment with oxygen plasma (EUROPLASMA, CD 600PC) at 100 watt, 120 mm Hg for 15 minutes until the uncovered part porous plastic became hydrophilic. The masks were then removed from the porous plastic sheet. The resulting porous plastic sheet had the discrete hydrophilic and hydrophobic regions. The regions that were covered by polyester tape masks remained hydrophobic and uncovered regions became hydrophilic.

### 6.5 MANUFACTURE OF DISCRETE HYDROPHILIC-HYDROPHOBIC POROUS MATERIALS BY SELECTIVE CORONA DISCHARGE TREATMENT

A sintered porous plastic sheet (T3, Porex Corporation, 0.4mm thick) was tightly covered with a polyester tape mask on both sides of porous plastic sheet. The covered porous plastic sheet was then exposed to corona discharge (Corotech) for a period of time until the uncovered part of the porous plastic became hydrophilic. The masks were then removed from the porous plastic. Discrete hydrophilic and hydrophobic regions resulted on the porous plastic. The regions that were covered by polyester tape remained hydrophobic, whereas uncovered regions became hydrophilic.

In another experiment, corona discharge was directly applied to porous plastic sheet without using a mask. The electrodes used in corona discharge were made in variety of shapes and the electrode shapes could be directly transferred to generate hydrophilic regions on porous plastic sheet by applying electrode in the right position. In this case, only parts of the porous plastic right underneath the electrodes became hydrophilic (Figure 5). A bar shaped corona electrode (0.5x5 cm, 100 watt) was positioned 1 mm above Porex porous plastic sheet and discharged for 10 seconds to make the part of the porous plastic that was directly underneath the electrode hydrophilic. The process is a stop and go process.

### 6.6 MANUFACTURE OF DISCRETE HYDROPHILIC-HYDROPHOBIC POROUS MATERIALS BY SELECTIVE ELECTRON BEAM TREATMENT

A sintered porous plastic sheet (T3, Porex Corporation, 0.4mm thick) is selectively exposed to electron beam for a period of time until the regions that are exposed the electron beam radiation become hydrophilic. The regions that are not exposed to the electron beam radiation remain hydrophobic. The geography of electron beam treatment determines the geographic shape of discrete hydrophilic-hydrophobic regions.

### 6.7 MANUFACTURE OF DISCRETE HYDROPHILIC-HYDROPHOBIC POROUS MATERIALS BY SELECTIVE PLASMA AND MULTI-LAYER COATING TREATMENT

A sintered porous plastic sheet (T3, Porex Corporation, 0.4mm thick) was tightly covered with a polyester tape mask on both sides of porous plastic sheet. The covered porous plastic sheet was then exposed to oxygen plasma (EUROPLASMA, CD 600PC) at 100 watt, 120 mm Hg for 15 minutes until the uncovered part porous plastic became hydrophilic. The masks were then removed from the porous plastic. The treated porous plastic had the discrete hydrophilic and hydrophobic regions. The regions that were covered by polyester tape remained hydrophobic and uncovered regions became hydrophilic.

The plasma treated discrete hydrophilic-hydrophobic porous plastic sheet was then immersed into 0.5 % of PEI (BASF, MW 750,000) ethanol-water solution for 10 minutes. The coated sheet was then rinsed with deionized water in an ultrasonic bath (VWR) at room temperature for 5 minutes. This washing was repeated three times. The treated sheet can be optionally dried in the air. The rinsed porous plastic sheet was then immersed into 0.5 % PAA (Aldrich, 523925, MW 250,000) ethanol-water solution for 10 minutes. The coated sheet was washed with 100 times volume of water in an ultrasonic bath (VWR) at room temperature for 5 minutes. This washing was repeated three times. The treated sheet was dried at room temperature. After the treatment, the regions that were exposed to the plasma became permanently hydrophilic and the regions that were not exposed to the plasma remained hydrophobic.

### 6.8 MANUFACTURE OF DISCRETE HYDROPHILIC-HYDROPHOBIC POROUS MATERIALS BY SELECTIVE CORONA DISCHARGE AND MULTI-LAYER COATING TREATMENTS

A sintered porous plastic sheet (T3, Porex Corporation, 0.4mm thick) was tightly covered with a polyester tape mask on both sides of porous plastic sheet. The covered porous plastic sheet was then exposed to corona discharge at 200 watt until the uncovered part of the porous plastic became hydrophilic. The masks were then removed from the porous plastic. The resulting porous plastic had the discrete hydrophilic and hydrophobic regions. The regions that were covered by polyester tape remained hydrophobic and uncovered regions became hydrophilic.

The corona discharge treated discrete hydrophilic-hydrophobic porous plastic sheet was then immersed into 0.5 % of PEI (BASF, MW 750,000) ethanol-water solution for 10 minutes. The coated sheet was then rinsed with deionized water in an ultrasonic bath (VWR) at room temperature for 5 minutes. This washing was repeated three times. The treated sheet can be optionally dried in the air. The rinsed porous plastic sheet was then immersed into 0.5 % PAA (Aldrich, 523925, MW 250,000) ethanol-water solution for 10 minutes. The coated sheet was washed with 100 times volume of water in an ultrasonic bath (VWR) at room temperature for 5 minutes. This washing was repeated three times. The treated sheet was dried at room temperature. After the treatment, the regions that were exposed to corona discharge became permanently hydrophilic and the regions that were not exposed to corona discharge remained hydrophobic.

### 6.9 MANUFACTURE OF DISCRETE HYDROPHILIC-HYDROPHOBIC POROUS MATERIALS BY SELECTIVE ELECTRON BEAM AND MULTI-LAYER COATING TREATMENTS

A sintered porous plastic sheet (T3, Porex Corporation, 0.4mm thick) is exposed to electron beam radiation until the porous plastic becomes hydrophilic. The untreated regions remain hydrophobic. The geography of electron beam treatment determines the geographic shape of discrete hydrophilic-hydrophobic regions.

The electron beam treated discrete hydrophilic-hydrophobic porous plastic sheet is then immersed into 0.5 % of PEI (BASF, MW 750,000) ethanol-water solution for 10 minutes. The coated sheet is then rinsed with deionized water in an ultrasonic bath (VWR) at room temperature for 5 minutes. This rinsing is repeated three times. The treated sheet can be optionally dried in the air. The rinsed porous plastic sheet is then immersed into 0.5 % PAA (Aldrich, 523925, MW 250,000) ethanol-water solution for 10 minutes. The coated sheet is washed with 100 times volume of water in an ultrasonic bath (VWR) at room temperature for 5 minutes. This washing is repeated three times. The treated sheet is dried at room temperature. After the treatment, the regions that are exposed to electron beam become permanently hydrophilic and the regions that are not exposed to electron beam remain hydrophobic.

### 6.10 MANUFACTURE OF DISCRETE HYDROPHOBIC-OLEOPHOBIC POROUS MATERIALS BY SELECTIVE PLASMA AND MULTI-LAYER COATING TREATMENTS

A sintered porous plastic sheet (T3, Porex Corporation, 0.4mm thick) was tightly covered with a polyester tape mask on both sides of porous plastic sheet. The covered porous plastic sheet was then exposed to oxygen plasma (EUROPLASMA, CD 600PC) at 100 watt, 120 mm Hg for 15 minutes until the uncovered part porous plastic became hydrophilic. The masks were then removed from the porous plastic. The resulting porous plastic had the discrete hydrophilic and hydrophobic regions. The regions that were covered by polyester tape remained hydrophobic and uncovered regions became hydrophilic.

Discrete hydrophilic and hydrophobic porous plastic sheet was then immersed into 0.5 % of PEI (BASF, MW 750,000) ethanol-water solution for 10 minutes. The coated sheet was then rinsed with deionized water in an ultrasonic bath (VWR) at room temperature for 5 minutes. This washing was repeated three times and the porous plastic sheet was optionally dried in the air. The hydrophilic regions of discrete porous plastic were then coated with PEI.

The PEI coated discrete porous plastic sheet was then immersed into 0.5 % of FSP fluorinated surfactants (DuPont) ethanol-water solution for 10 minutes. The coated sheet was washed with deionized water in an ultrasonic bath (VWR) at room temperature for 5 minutes. This washing was repeated three times. The treated sheet was dried at room temperature. The PEI coated regions, which were hydrophilic after the plasma treatment then became oleophobic.

### 6.11 MANUFACTURE OF DISCRETE HYDROPHOBIC-OLEOPHOBIC POROUS MATERIALS BY SELECTIVE CORONA DISCHARGE AND MULTI-LAYER COATING TREATMENTS

A sintered porous plastic sheet (T3, Porex Corporation, 0.4mm thick) was tightly covered with a polyester tape mask on both sides of porous plastic sheet. The covered porous plastic sheet was then exposed to corona discharge at 200 watt until the uncovered part of the porous plastic became hydrophilic. The masks were then removed from the porous plastic. The resulting porous plastic had the discrete hydrophilic and hydrophobic regions. The regions that were covered by polyester tape remained hydrophobic and uncovered regions became hydrophilic.

The corona discharge treated discrete hydrophilic-hydrophobic porous plastic sheet was then immersed into 0.5 % of PEI (BASF, MW 750,000) ethanol-water solution for 10 minutes. The coated sheet was then rinsed with deionized water in an ultrasonic bath (VWR) at room temperature for 5 minutes. This washing was repeated three times. The treated sheet can be optionally dried in the air. The rinsed porous plastic sheet was then immersed into 0.5 % of FSP fluorinated surfactants (DuPont) ethanol-water solution for 10 minutes. The coated sheet was washed with deionized water in an ultrasonic bath (VWR) at room temperature for 5 minutes. This washing was repeated three times. The treated sheet was dried at room temperature. The PEI coated regions, which were hydrophilic after corona discharge treatment then became oleophobic.

### 6.12 MANUFACTURE OF DISCRETE HYDROPHOBIC-HYDROPHILIC AND DISCRETE CHARGED POROUS MATERIALS BY SELECTIVE PLASMA AND MULTI-LAYER COATING TREATMENTS

After porous plastic sheet was pre-activated by plasma discharge as disclosed in example 6.4, certain hydrophilic regions (Region As) were treated with positively charged polyelectrolyte solution (*i*.*e*., 0.5 % of PEI (BASF, MW 750,000) ethanol-water solution for 10 minutes), and other hydrophilic regions (Region Bs) were treated with negatively charged polyelectrolyte solution (*i.e.,* 0.5 % PAA (Aldrich, 523925, MW 250,000) ethanol-water solution for 10 minutes). The discrete porous plastic sheet was then washed and dried. The region As were positively charged (positive ξ potential); the region Bs were negatively charged (negative ξ potential); and hydrophobic regions on discrete porous materials were neutral at neutral pH condition.

Region As, which were treated with positively charged polyelectrolyte solution (*i*.*e*., PEI) can then be sequentially treated with a negatively charged polyelectrolyte solution (*i*.*e*., PAA). The region As became negatively charged (negative ξ potential). In a similar way, Region Bs, which were treated with negatively charged polyelectrolyte solution (*i*.*e*., PAA) could then be sequentially treated with a positively charged polyelectrolyte solution (*i*.*e*., PEI). The region Bs became positively charged (positive ξ potential). The above selective polyelectrolyte solution treatment can be repeated several times until the desired film thickness or charge density is achieved.

### 6.13 MANUFACTURE OF DISCRETE HYDROPHOBIC-HYDROPHILIC AND DISCRETE CHARGED POROUS MATERIALS BY SELECTIVE CORONA DISCHARGE AND MULTI-LAYER COATING TREATMENTS

After porous plastic sheet was pre-activated by corona discharge as disclosed in examples 6.5, certain hydrophilic regions (Region As) were treated with positively charged polyelectrolyte solution (*i*.*e*., 0.5 % of PET (BASF, MW 750,000) ethanol-water solution for 10 minutes), and other hydrophilic regions (Region Bs) were treated with negatively charged polyelectrolyte solution (*i*.*e*., 0.5 % PAA (Aldrich, 523925, MW 250,000) ethanol-water solution for 10 minutes). The discrete porous plastic sheet was then washed and dried. The region As were positively charged (positive ξ potential); the region Bs were negatively charged (negative ξ potential); and hydrophobic regions on discrete porous materials were neutral at neutral pH condition.

Region As, which were treated with positively charged polyelectrolyte solution (*i*.*e*., PEI) can then be sequentially treated with a negatively charged polyelectrolyte solution (*i*.*e*., PAA). Region As became negatively charged (negative ξ potential). In a similar way, Region Bs, which were treated with negatively charged polyelectrolyte solution (*i*.*e*., PAA) could then be sequentially treated with a positively charged polyelectrolyte solution (*i*.*e*., PEI). Region Bs became positively charged (positive ξ potential). The above selective polyelectrolyte solution treatment can be repeated several times until the desired film thickness or charge density is achieved.

### 6.14 MANUFACTURE OF POROUS MATERIALS CONTAINING DISCRETE HYDROPHOBIC-HYDROPHILIC REGIONS AND DISCRETE CHEMICAL FUNCTIONAL GROUPS BY SELECTIVE PLASMA AND MULTI-LAYER COATING TREATMENTS

After porous plastic sheet was pre-activated by plasma discharge as disclosed in example 6.4, certain hydrophilic regions (Region As) were treated with polyelectrolytes that contain amino-functional groups (*i*.*e*., 0.5 % of PEI (BASF, MW 750,000) ethanol-water solution for 10 minutes), and other hydrophilic regions (Region Bs) were treated with polyelectrolytes that contain carboxylic functional groups (*i*.*e*., 0.5 % PAA (Aldrich, 523925, MW 250,000) ethanol-water solution for 10 minutes). The discrete porous plastic sheet was then washed and dried. The region As were amino group functionalized; the region Bs were carboxylic acid group functionalized; and hydrophobic regions are chemically inert with hydrocarbon backbones.

Region As, which were treated with amino functional polyelectrolyte (*i*.*e*., PEI) can then be sequentially treated with a negatively charged molecules that contains other functional groups to provide region As with variety of functional groups. PEI coated regions can then be treated with: PAA to provide carboxylic acid groups; poly(vinyl sulfate) to provide sulfate groups; phosphate containing molecules to provide phosphate groups (*i*.*e*., lipids, molecules containing both phosphate and carboxylic acid); biotin containing negative charge molecules to provide biotin functional groups (*i*.*e*., carboxylic functional biotin, Shearwater); thiol containing negative charge molecules to provide thiol groups (*i.e.,* β-mercaptoacetic acid); or peptides that have free carboxylic acid groups; or nucleic acids containing phosphate groups (*i.e.,* DNA and RNA fragments containing phosphate groups).

In a similar way, the region Bs, which were treated with negatively charged polyelectrolyte solution (*i*.*e*., PAA) can then be sequentially treated with functional groups or molecules that contain positive charges to provide region Bs with variety of functional groups.

### 6.15 MANUFACTURE OF POROUS MATERIALS CONTAINING DISCRETE HYDROPHILIC-HYDROPHOBIC REGIONS AND DISCRETE CHEMICAL FUNCTIONAL GROUPS BY SELECTIVE CORONA DISCHARGE AND MULTI-LAYER COATING TREATMENT

After porous plastic sheet was pre-activated by corona discharge as disclosed in examples 6.5, certain hydrophilic regions (Region As) were treated with polyelectrolytes that contain amino-functional groups, (*i*.*e*., 0.5 % of PET (BASF, MW 750,000) ethanol-water solution for 10 minutes), and other hydrophilic regions (Region Bs) were treated with polyelectrolytes that contain carboxylic functional groups, (*i*.*e*., 0.5 % PAA (Aldrich, 523925, MW 250,000) ethanol-water solution for 10 minutes). The discrete porous plastic sheet was then washed and dried. The region As were amino group functionalized; the region Bs were carboxylic acid group functionalized; and hydrophobic regions are chemically inert with hydrocarbon backbones.

Region As, which were treated with amino functional polyelectrolyte (*i*.*e*., PEI) can then be sequentially treated with a negatively charged molecules that contains other functional groups to provide region As with variety of functional groups. PEI coated regions can then be treated with: PAA to provide carboxylic acid groups; poly(vinyl sulfate) to provide sulfate groups; phosphate containing molecules to provide phosphate groups (*i*.*e*., lipids, molecules containing both phosphate and carboxylic acid); biotin containing negative charge molecules to provide biotin functional groups (*i*.*e*., carboxylic functional biotin, Shearwater); thiol containing negative charge molecules to provide thiol groups (*i*.*e*., β**-**mercaptoacetic acid); or peptides that have free carboxylic acid groups; or nucleic acids containing phosphate groups (*i*.*e*., DNA and RNA fragments containing phosphate groups).

In a similar way, the region Bs, which were treated with negatively charged polyelectrolyte solution (*i*.*e*., PAA) can then be sequentially treated with functional groups or molecules that contain positive charges to provide region Bs with variety of functional groups.

## Claims

1. A porous material comprising a polymeric substrate comprising a surface, the surface comprising a plurality of discrete hydrophilic regions coated with a first layer of polyelectrolyte molecules, wherein the polyelectrolyte molecules are bound to the discrete hydrophilic regions by a plurality of covalent and electrostatic interactions.

2. The material of claim 1, wherein the polymeric substrate comprises a polyolefin, polyether, polyurethane, polycarbonate, polyetheretherketone, poly (phenylene oxide), poly (ether sulfone), polysulfone, nitrocellulose, cellulose, fluorinated polymers, PTFE, porous fiber materials or nylon.

3. The material of claim 2, wherein the polyolefin comprises ethylene vinyl acetate, ethylene methyl acrylate, polyethylene, polypropylene, ethylene-propylene rubber, ethylene-propylene-diene rubbers, poly(1-butene), polystyrene, poly (2-butene), poly(1-pentene), poly(2-pentene), poly(3-methyl-1-pentene), poly(4-methyl-1-pentene), 1,2-poly-1, 3-butadiene, 1,4-poly-1, 3-butadiene; polyisoprene, polychloroprene, poly(vinyl acetate), poly(vinylidene chloride), poly(vinylidene fluoride), poly(tetrafluoroethylene), or mixtures thereof.

4. The material of any one of claims 1-3, further comprising a second layer of polyelectrolyte molecules bound to the first layer through covalent and electrostatic interactions.

5. The material of claim 1-4, wherein the polyelectrolyte molecules of the first layer and/or the second layer are independently selected from the group consisting of polyethyleneimide, poly(vinylimidazoline), quaterized polyacrylamide, carboxyl modified polyacrylamide, polyvinylpyridine, poly(vinylpyrrolidone), polyvinylamine, polyallylamine, chitosan, polylysine, poly(acrylate trialkyl ammonia salt ester), cellulose, poly(acrylic acid), polymethylacrylic acid, poly(maleic acid), poly(styrenesulfonic acid), poly(vinylsulfonic acid), poly(toluene sulfonic acid), poly(methyl vinyl ether-alt-maleic acid), poly(glutamic acid), surfactant, dextran sulfate, hyaluronic acid, heparin, alginic acid, adipic acid, chemical dye, protein, enzyme, nucleic acid, peptide, or a salt, ester or copolymer thereof.

6. The material of claim 4 or 5, further comprising one or more additional layers, wherein each additional layer is bound to the layer below it through covalent bonds, electrostatic interactions or combinations thereof.

7. The material of claim 6, wherein the one or more additional layers comprises a polyelectrolyte, neutral polymer, small molecule, biomolecule, or combination thereof.

8. The material of claim 7, wherein the neutral polymer comprises an isocyanated terminated polymer, epoxy terminated polymer, or hydroxylsuccinimide terminated polymer.

9. The material of claim 7 or 8, wherein the neutral polymer comprises polyurethane, poly(ethylene glycol), or polysiloxane.

10. The material of claim 7, wherein the small molecule comprises dodecyltrimethylammonium bromide, phosphate, sulfonate, bromate, dye, lipid, metal ion, or fluorinated surfactant.

11. The material of claim 7, wherein the biomolecule comprises a protein, enzyme, hormone, peptide, nucleic acid, oligonucleotide, or polysaccharide.

12. The material of any one of claims 1-11, wherein the plurality of discrete hydrophilic regions comprises 96 hydrophilic regions.

13. The material of any one of claims 1-12, wherein each of the of the discrete hydrophilic regions has a circular shape.

14. A single-pieced porous polymeric material comprising a surface, the surface comprising a plurality of discrete hydrophilic regions coated with polyelectrolyte molecules, wherein the polyelectrolyte molecules comprise a plurality of functional groups and are bound to the discrete hydrophilic regions by a plurality of covalent and electrostatic interactions.

15. The material of claim 14, wherein the plurality of functional groups have electrical charges.

16. The material of claim 15, wherein the electrical charges comprise positive, charges, negative charges, or combinations thereof.

17. The material of any one of claims 14-16, wherein the plurality of functional groups are selected from the group consisting of amino functionalities, carboxylic acid functionalities, sulfate functionalities, polysaccharides, surfactants, fluorinated surfactants and biotin.

18. The material of any one of claims 14-17, wherein the functional groups are the same.

19. The material of any one of claims 14-17, wherein the functional groups are different.

20. The material of any one of claims 14-19, wherein the polyelectrolyte molecules comprise polyethyleneimide, poly(vinylimidazoline), quaterized polyacrylamide, carboxyl modified polyacrylamide, polyvinylpyridine, poly(vinylpyrrolidone), polyvinylamine, polyallylamine, chitosan, polylysine, poly(acrylate trialkyl ammonia salt ester), cellulose, poly(acrylic acid), polymethylacrylic acid, poly(maleic acid), poly(styrenesulfonic acid), poly(vinylsulfonic acid), poly(toluene sulfonic acid), poly(methyl vinyl ether-alt-maleic acid), poly(glutamic acid), surfactant, dextran sulfate, hyaluronic acid, heparin, alginic acid, adipic acid, chemical dye, protein, enzyme, nucleic acid, peptide, or a salt, ester or copolymer thereof.

21. A method of making porous materials comprising a plurality of discrete hydrophilic regions comprising:
masking a porous polymeric substrate to produce a patterned surface comprising masked regions and unmasked regions;
activating the unmasked regions; and
coating the activated unmasked regions with a first layer of polyelectrolyte molecules, wherein the polyelectrolyte molecules are bound to the activated unmasked regions by covalent and electrostatic interactions.

22. The method of claim 21, wherein activating comprises exposing the unmasked regions to plasma activation, corona discharge, electron beam, or ultraviolet radiation.

23. The method of claim 21 or 22, further comprising coating the activated unmasked regions with a second layer of polyelectrolyte molecules, wherein the second layer of polyelectrolyte molecules are bound to the first layer through covalent and electrostatic interactions.

24. The method of claim 21-23, wherein the polyelectrolyte molecules of the first layer and/or the second layer are independently selected from the groups consisting of polyethyleneimide, poly(vinylimidazoline), quaterized polyacrylamide, carboxyl modified polyacrylamide, polyvinylpyridine, poly(vinylpyrrolidone), polyvinylamine, polyallylamine, chitosan, polylysine, poly(acrylate trialkyl ammonia salt ester), cellulose, poly(acrylic acid), polymethylacrylic acid, poly(maleic acid), poly(styrenesulfonic acid), poly(vinylsulfonic acid), poly(toluene sulfonic acid), poly(methyl vinyl ether-alt-maleic acid), poly(glutamic acid), surfactant, dextran sulfate, hyaluronic acid, heparin, alginic acid, adipic acid, chemical dye, protein, enzyme, nucleic acid, peptide, or a salt, ester or copolymer thereof.

25. The method of claim 23 or 24, further comprising coating the activated unmasked regions with one or more additional layers, wherein each additional layer is bound to the layer below it through covalent bonds, electrostatic interactions, or combinations thereof.

26. The method of claim 25, wherein the one or more additional layers comprises a polyelectrolyte, neutral polymer, small molecule, biomolecule, or combination thereof.

27. The method of claim 26, wherein the biomolecule comprises a protein, enzyme, hormone, peptide, nucleic acid, oligonucleotide, nucleic acid, or polysaccharide.

28. A method for making porous materials comprising a plurality of discrete hydrophilic regions comprising:
providing a porous substrate;
disposing a polymer powder in a pattern on the porous substrate; and
compress sintering the porous substrate and powder.

29. The method of claim 28, wherein the porous substrate comprises a polyolefin, polyether, polyurethane, polycarbonate, polyetheretherketone, poly(phenylene oxide), poly(ether sulfone), polysulfone, nitrocellulose, cellulose, fluorinated polymers, PTFE, porous fiber materials or nylon.

30. The method of claim 29, wherein the polyolefin is selected from a group consisting of ethylene vinyl acetate, ethylene methyl acrylate, polyethylene, polypropylene, ethylene-propylene rubber, ethylene-propylene-diene rubbers, poly(1-butene), polystyrene, poly (2-butene), poly(1-pentene), poly(2-pentene), poly(3-methyl-1-pentene), poly(4-methyl-1-pentene), 1,2-poly-1, 3-butadiene, 1,4-poly-1, 3-butadiene; polyisoprene, polychloroprene, poly(vinyl acetate), poly(vinylidene chloride), poly(vinylidene fluoride), poly(tetrafluoroethylene), or mixtures thereof.

31. The method of any one of claims 28-30, wherein the polymer powder comprises a plurality of different polymers.

32. The method of any one of claims 28-31, further comprising coating the plurality of discrete hydrophilic regions with a first layer of polyelectrolyte molecules, wherein the polyelectrolyte molecules are bound to the discrete hydrophilic regions by covalent and electrostatic interactions.

33. The method of claim 32 further comprising coating the plurality of discrete hydrophilic regions with a second layer of polyelectrolyte molecules, wherein the second layer of polyelectrolyte molecules are bound to the first layer by covalent and electrostatic interactions.

34. The method of claim 32 or 33, wherein the polyelectrolyte molecules of the first layer and/or the second layer are independently selected from the group consisting of polyethyleneimide, poly(vinylimidazoline), quaterized polyacrylamide, carboxyl modified polyacrylamide, polyvinylpyridine, poly(vinylpyrrolidone), polyvinylamine, polyallylamine, chitosan, polylysine, poly(acrylate trialkyl ammonia salt ester), cellulose, poly(acrylic acid), polymethylacrylic acid, poly(maleic acid), poly(styrenesulfonic acid), poly(vinylsulfonic acid), poly(toluene sulfonic acid), poly(methyl vinyl ether-alt-maleic acid), poly(glutamic acid), surfactant, dextran sulfate, hyaluronic acid, heparin, alginic acid, adipic acid, chemical dye, protein, enzyme, nucleic acid, peptide, or a salt, ester or copolymer thereof.

35. The method of claim 33 or 34, further comprising coating the plurality of discrete hydrophilic regions with one or more additional layers, wherein each additional layer is bound to the layer below it through covalent bonds, electrostatic interactions, or combinations thereof.

36. The method of claim 35, wherein the one or more additional layers comprises a polyelectrolyte, neutral polymer, small molecule, biomolecule, or combination thereof.

37. The method of claim 36, wherein the small molecule comprises sodium dodecylsulfate, dodecyltrimethylammonium bromide, phosphate, sulfonate, bronate, dye, lipid, metal ion, or fluorinated surfactant.

38. The method of claim 36, wherein the biomolecule comprises a protein, enzyme, hormone, peptide, nucleic acid, oligonucleotide, or polysaccharide.

## Patentansprüche

1. Poröses Material umfassend einen polymeren Träger umfassend eine Oberfläche, wobei die Oberfläche eine Vielzahl von diskreten hydrophilen Bereichen umfasst, welche mit einer ersten Schicht von Polyelektrolytmolekülen beschichtet sind, wobei die Polyelektrolytmoleküle an die diskreten hydrophilen Bereiche durch eine Vielzahl von kovalenten und elektrostatischen Wechselwirkungen gebunden sind.

2. Das Material nach Anspruch 1, wobei der polymere Träger ein Polyolefin, Polyether, Polyurethan, Polycarbonat, Polyetheretherketon, Poly(phenylerioxid), Poly(ethersulfon), Polysulfon, Nitrozellulose, Zellulose, fluorierte Polymere, PTFE, poröse Fasermaterialien oder Nylon umfasst.

3. Das Material nach Anspruch 2, wobei das Polyolefin Ethylenvinylacetat, Ethylenmethylacrylat, Polyethylen, Polypropylen, Ethylenpropylen-Gummi, Ethylen-Propylen-Dien-Gummis, Poly(1-buten), Polystyrol, Poly(2-buten), Poly(1-penten), Poly(2-penten), Poly(3-methyl-1-penten), Poly(4-methyl-1-penten), 1,2-Poly-1,3-butadien, 1,4-Poly-1,3-butadien, Polyisopren, Polychloropren, Poly(vinylacetat), Poly(vinylidenchlorid), Poly(vinylidenfluorid), Poly(tetrafluorethylen), oder Gemische davon umfasst.

4. Das Material nach einem der Ansprüche 1-3, ferner umfassend eine zweite Schicht von Polyelektrolytmolekülen, gebunden an die erste Schicht durch kovalente und elektrostatische Wechselwirkungen.

5. Das Material nach einem der Ansprüche 1-4, wobei die Polyelektrolytmoleküle der ersten Schicht und/oder der zweiten Schicht unabhängig ausgewählt sind aus der Gruppe bestehend aus Polyethylenimid, Poly(vinylimidazolin), quaternisiertes Polyacrylamid, Carboxyl-modifiziertes Polyacrylamid, Polyvinylpyridin, Poly(vinylpyrrolidon), Polyvinylamin, Polyallylamin, Chitosan, Polylysin, Poly(acrylattrialkylammoniumsalzester), Zellulose, Poly(acrylsäure), Polymethacrylsäure, Poly(maleinsäure), Poly(styrolsulfonsäure), Poly(vinylsulfonsäure), Poly(toluolsulfonsäure), Poly(methylvinylethersalz-Maleinsäure), Poly(glutaminsäure), oberflächenaktives Mittel, Dextransulfat, Hyaluronsäure, Heparin, Alginsäure, Adipinsäure, chemischer Farbstoff, Protein, Enzym, Nukleinsäure, Peptid, oder einem Salz, Ester oder Co-Polymer davon.

6. Das Material nach Anspruch 4 oder 5, ferner umfassend eine oder mehrere zusätzliche Schichten, wobei jede zusätzliche Schicht an die darunterliegende Schicht durch kovalente Bindungen, elektrostatische Wechselwirkungen oder Kombinationen davon, gebunden ist.

7. Das Material nach Anspruch 6, wobei die eine oder mehreren zusätzlichen Schichten ein Polyelektrolyt, Neutralpolymer, kleines Molekül, Biomolekül oder Kombinationen davon, umfasst/umfassen.

8. Das Material nach Anspruch 7, wobei das Neutralpolymer ein Polymer mit Isocyanatende, ein Polymer mit Epoxyende oder ein Polymer mit Hydroxylsuccinimidende umfasst.

9. Das Material nach Anspruch 7 oder 8, wobei das Neutralpolymer Polyurethan, Poly(ethylenglykol) oder Polysiloxan umfasst.

10. Das Material nach Anspruch 7, wobei das kleine Molekül Dodecyltrimethylammoniumbromid, Phosphat, Sulfonat, Bromat, Farbstoff, Lipid, Metallion oder fluoriertes oberflächenaktives Mittel umfasst.

11. Das Material nach Anspruch 7, wobei das Biomolekül ein Protein, Enzym, Hormon, Peptid, Nukleinsäure, Oligonukleotid, oder Polysaccharid umfasst.

12. Das Material nach einem der Ansprüche 1-11, wobei die Vielzahl an diskreten hydrophilen Bereichen 96 hydrophile Bereiche umfasst.

13. Das Material nach einem der Ansprüche 1-12, wobei jeder der diskreten hydrophilen Bereiche eine kreisförmige Form aufweist.

14. Einstückiges poröses polymeres Material, umfassend eine Oberfläche, wobei die Oberfläche eine Vielzahl von diskreten hydrophilen Bereichen umfasst, welche mit Polyelektrolytmolekülen beschichtet sind, wobei die Polyelektrolytmoleküle eine Vielzahl von funktionellen Gruppen aufweisen und an die diskreten hydrophilen Bereiche durch eine Vielzahl von kovalenten und elektrostatischen Wechselwirkungen gebunden sind.

15. Das Material nach Anspruch 14, wobei die Vielzahl von funktionellen Gruppen elektrische Ladungen aufweist.

16. Das Material nach Anspruch 15, wobei die elektrischen Ladungen positive Ladungen, negative Ladungen oder Kombinationen davon umfassen.

17. Das Material nach einem der Ansprüche 14-16, wobei die Vielzahl an funktionellen Gruppen ausgewählt sind aus der Gruppe bestehend aus Aminofunktionalitäten, Carbonsäurefunktionalitäten, Sulfatfunktionalitäten, Polysacchariden, oberflächenaktiven Mitteln, fluorierten oberflächenaktiven Mitteln und Biotin.

18. Das Material nach einem der Ansprüche 14-17, wobei die funktionellen Gruppen die gleichen sind.

19. Das Material nach einem der Ansprüche 14-17, wobei die funktionellen Gruppen unterschiedlich sind.

20. Das Material nach einem der Ansprüche 14-19, wobei die Polyelektrolytmoleküle Polyethylenimid, Poly(vinylimidazolin), quaternisiertes Polyacrylamid, Carboxyl-modifiziertes Polyacrylamid, Polyvinylpyridin, Poly(vinylpyrrolidon), Polyvinylamin, Polyallylamin Chitosan, Polylysin, Poly(acrylattrialkylammoniumsalzester), Zellulose, Poly(acrylsäure), Polymethacrylsäure, Poly(maleinsäure), Poly(styrolsulfonsäure), Poly(vinylsulfonsäure), Poly(toluolsulfonsäure), Poly(methylvinylethersalz-Maleinsäure), Poly(glutaminsäure), oberflächenaktives Mittel, Dextransulfat, Hyaluronsäure, Heparin, Alginsäure, Adipinsäure, chemischer Farbstoff, Protein, Enzym, Nukleinsäure, Peptid oder ein Salz; Ester oder Co-Polymer davon umfassen.

21. Verfahren zur Herstellung eines porösen Materials umfassend eine Vielzahl von diskreten hydrophilen Bereichen umfassend:
Maskieren eines porösen polymeren Trägers zur Erzeugung einer gemusterten Oberfläche umfassend maskierte Bereiche und unmaskierte Bereiche;
Aktivieren der unmaskierten Bereiche; und
Beschichten der aktivierten unmaskierten Bereiche mit einer ersten Schicht von Polyelektrolytmolekülen, wobei die Polyelektrolytmoleküle gebunden sind an die aktivierten unmaskierten Bereiche durch kovalente und elektrostatische Wechselwirkungen.

22. Das Verfahren nach Anspruch 21, wobei Aktivieren umfasst Exponieren der unmaskierten Bereiche gegenüber Plasmaaktivierung, Coronaentladung, Elektronenstrahl oder ultravioletter Bestrahlung.

23. Das Verfahren nach Anspruch 21 oder 22, ferner umfassend Beschichten der aktivierten unmaskierten Bereiche mit einer zweiten Schicht von Polyelektrolytmolekülen, wobei die zweite Schicht von Polyelektrolytmolekülen gebunden ist an die erste Schicht durch kovalente und elektrostatische Wechselwirkungen.

24. Das Verfahren nach Anspruch 21-23, wobei die Polyelektrolytmoleküle der ersten Schicht und/oder der zweiten Schicht unabhängig ausgewählt sind aus der Gruppe bestehend aus Polyethylenimid, Poly(vinylimidazolin), quaternisiertes Polyacrylamid, Carboxyl-modifiziertes Polyacrylamid, Polyvinylpyridin, Poly(vinylpyrrolidon), Polyvinylamin, Polyallylamin, Chitosan, Polylysin, Poly(acrylattrialkylammoniumsalzester), Zellulose, Poly(acrylsäure), Polymethacrylsäure, Poly(maleinsäure), Poly(styrolsulfonsäure), Poly(vinylsulfonsäure), Poly(toluolsulfonsäure), Poly(methylvinylethersalz-Maleinsäure), Poly(glutaminsäure), oberflächenaktives Mittel, Dextransulfat, Hyaluronsäure, Heparin, Alginsäure, Adipinsäure, chemischer Farbstoff, Protein, Enzym, Nukleinsäure, Peptid, oder einem Salz, Ester oder Co-Polymer davon.

25. Das Verfahren nach Anspruch 23 oder 24, ferner umfassend Beschichten der aktivierten unmaskierten Bereiche mit einer oder mehreren zusätzlichen Schichten, wobei jede zusätzliche Schicht an die unterliegende Schicht gebunden ist durch kovalente Bindungen, elektrostatische Wechselwirkungen oder Kombinationen davon.

26. Das Verfahren nach Anspruch 25, wobei die eine oder mehreren zusätzlichen Schichten umfassen ein Polyelelktrolyt, Neutralpolymer, kleines Molekül, Biomolekül oder eine Kombination davon.

27. Das Verfahren nach Anspruch 26, wobei das Biomolekül umfasst ein Protein, Enzym, Hormon, Peptid, Nukleinsäure, Oligonukleotid, Nukleinsäure oder Polysaccharid.

28. Verfahren zur Herstellung porösen Materials umfassend eine Vielzahl von diskreten hydrophilen Bereichen umfassend:
Bereitstellen eines porösen Trägers;
Abscheiden eines Polymerpulvers in Mustern auf dem porösen Träger; und
Verdichtungssintern des porösen Trägers und des Pulvers.

29. Das Verfahren nach Anspruch 26, wobei der poröse Träger umfasst ein Polyolefin, Polyether, Polyurethan, Polycarbonat, Polyetheretherketon, Poly(phenylenoxid), Poly(ethersulfon), Polysulfon, Nitrozellulose, Zellulose, fluorierte Polymere, PTFE, poröse Fasermaterialien oder Nylon.

30. Das Verfahren nach Anspruch 29, wobei das Polyolefin ausgewählt ist aus der Gruppe bestehend aus Ethylenvinylacetat, Ethylenmethylacrylat, Polyethylen, Polypropylen, Ethylenpropylen-Gummi, Ethylen-Propylen-Dien-Gummies, Poly(1-buten), Polystyrol, Poly(2-buten), Poly(1-penten), Poly(2-penten), Poly(3-methyl-1-penten), Poly(4-methyl-1-penten), 1,2-Poly-1,3-butadien, 1 ,4-Poly-1 ,3-butadien; Polyisopren, Polychloropren, Poly(vinylacetat), Poly(vinylidenchlorid), Poly(vinylidenfluorid), Poly(tetrafluorethylen), oder Gemische davon.

31. Das Verfahren nach einem der Ansprüche 28-30, wobei das Polymerpulver eine Vielzahl unterschiedlicher Polymere umfasst.

32. Das Verfahren nach einem der Ansprüche 28-31, ferner umfassend Beschichten der Vielzahl von diskreten hydrophilen Bereichen mit einer ersten Schicht von Polyelektrolytmolekülen, wobei die Polyelektrolytmoleküle gebunden sind an die diskreten hydrophilen Bereiche durch kovalente und elektrostatische Wechselwirkungen.

33. Das Verfahren nach Anspruch 32, ferner umfassend Beschichten der Vielfalt an diskreten hydrophilen Bereichen mit einer zweiten Schicht von Polyelektrolytmolekülen, wobei die zweite Schicht von Polyelektrolytmolekülen gebunden ist an die erste Schicht durch kovalente und elektrostatische Wechselwirkungen.

34. Das Verfahren nach Anspruch 32 oder 33, wobei die Polyelektrolytmoleküle der ersten Schicht und/oder der zweiten Schicht unabhängig ausgewählt sind aus der Gruppe bestehend aus Polyethylenimid, Poly(vinylimidazolin), quaternisiertes Polyacrylamid, Carboxyl-modifiziertes Polyacrylamid, Polyvinylpyridin, Poly(vinylpyrrolidon), Polyvinylamin, Polyallylamin, Chitosan, Polylysin, Poly(acrylattrialkylammoniumsalzester), Zellulose, Poly(acrylsäure), Polymethacrylsäure, Poly(maleinsäure), Poly(styrolsulfonsäure), Poly(vinylsulfonsäure), Poly(toluolsulfonsäure), Poly(methylvinylethersalz-Maleinsäure), Poly(glutaminsäure), oberflächenaktives Mittel, Dextransulfat, Hyaluronsäure, Heparin, Alginsäure, Adipinsäure, chemischer Farbstoff, Protein, Enzym, Nukleinsäure, Peptid, oder ein Salz, Ester oder Co-Polymer davon.

35. Das Verfahren nach Anspruch 33 oder 34, ferner umfassend Beschichten der Vielzahl an diskreten hydrophilen Bereichen mit einer oder mehreren zusätzlichen Schichten, wobei jede zusätzliche Schicht gebunden ist an die darunterunterliegende Schicht durch kovalente Bindungen, elektrostatische Wechselwirkungen oder Kombinationen davon.

36. Das Verfahren nach Anspruch 35, wobei die eine oder mehreren zusätzlichen Schichten ein Polyelektrolyt, Neutralpolymer, kleines Molekül, Biomolekül oder Kombinationen davon umfasst/umfassen.

37. Das Verfahren nach Anspruch 36, wobei das kleine Molekül umfasst Natriumdodecylsulfat, Dodecyltrimethylammoniumbromid, Phosphat, Sulfonat, Bromat, Farbstoff, Lipid, Metallion oder fluoriertes oberflächenaktives Mittel.

38. Das Verfahren nach Anspruch 36, wobei das Biomolekül umfasst ein Protein, Enzym, Hormon, Peptid, Nukleinsäure, Oligonukleotid oder Polysaccharid.

## Revendications

1. Matériau poreux comprenant un substrat polymère ayant une surface comprenant un ensemble de zones hydrophobes discrètes revêtues d'une première couche de molécules polyélectrolytiques, ces molécules polyélectrolytiques étant liées aux régions hydrophiles discrètes par un ensemble de liaisons covalentes et d'interactions électrostatiques.

2. Matériau selon la revendication1,
dans lequel
le substrat polymère comporte l'un des composés suivants :
polyoléfine, polyéther, polyuréthane, polycarbonate, polyétheréthercétone, poly(phénylène oxyde), poly(éther sulfone), polysulfone, nitrocellulose, cellulose, polymères fluorés, PTFE, matériaux fibreux poreux ou nylon.

3. Matériau selon la revendication 2,
dans lequel
la polyoléfine comporte l'un des composés suivants :
éthylène vinylacétate, éthylène méthyl acrylate, polyéthylène, polypropylène, caoutchouc éthylène-propylène, caoutchoucs éthylène-propylène-diène, poly(1-butène), polystyrène, poly(2-butène), poly(1-pentène), poly(2-pentène), poly(3-méthyl-1-pentène), poly(4-méthyl-1-pentène), 1,2-poly-1,3-butadiène, 1,4-poly-1,3-butadiène, polyisoprène, polychloroprène, polyvinylacétate, polychlorure de vinylidène, polyfluorure de vinylidène, polytétrafluoroéthylène, ou leurs mélanges.

4. Matériau selon l'une des revendications 1 à 3,
comportant en outre une seconde couche de molécules polyélectrolytiques reliée à la première couche par des liaisons covalentes et des interactions électrostatiques.

5. Matériau selon l'une des revendications 1 à 4,
dans lequel les molécules polyélectrolytiques de la première couche et/ou de la seconde couche, sont choisies de manière indépendante dans le groupe formé par les composés suivants:
polyéthylèneimide, polyvinylimidazoline, polyacrylamide quaternisé, polyacrylamide modifié par des groupes carboxyle, polyvinyle pyridine, polyvinylpyrrolidone, polyvinylamine, poly(allylamine), chitosane, polylysine, ester sel de polyacrylate trialkyl ammonium, cellulose, acide polyacrylique, acide polyméthylacrylique, acide polymaléique, acide polystyrène sulfonique, acide polyvinyle sulfonique, acide polytoluène sulfonique, poly(méthyl vinyle éther-alt-acide-maléique), acide polyglutamique, agents tensioactif/ sulfate de dextrane, acide hyaluronique, héparine, acide alginique, acide adipique, colorant chimique, protéine, enzyme, acide nucléique, peptide, ou leurs sels, esters ou copolymères.

6. Matériau selon la revendication 4 ou 5,
comprenant en outre au moins une couche supplémentaire, cette ou chacune de ces couche(s) supplémentaire(s) étant liée(s) à la couche située au dessous par des liaisons covalentes, des interactions électrostatiques ou leurs combinaisons.

7. Matériau selon la revendication 6,
**caractérisé en ce que**
la ou les couche(s) supplémentaire(s) comporte(nt) un polyélectrolyte, un polymère neutre, une petite molécule, une biomolécule, ou leurs combinaisons.

8. Matériau selon la revendication 7,
dans lequel
le polymère neutre comporte un polymère ayant un groupe isocyano terminal, un groupe époxy terminal, ou un groupe hydroxylsuccinimide terminal.

9. Matériau selon la revendication 7 ou 8,
dans lequel
le polymère neutre comporte du polyuréthane, du poly(éthylène glycol) ou du polysiloxane.

10. Matériau selon la revendication 7,
dans lequel
la petite molécule comporte du bromure de dodécyltriméthylammonium, un phosphate, un sulfonate, un bromate, un colorant, un lipide, un ion métallique, ou un agent tensioactif fluoré.

11. Matériau selon la revendication 7,
dans lequel
la biomolécule comporte une protéine, une enzyme, une hormone, un peptide, un acide nucléique, un oligonucléotide ou un polysaccharide.

12. Matériau selon l'une des revendications 1 à 11,
dans lequel
l'ensemble des régions hydrophiles discrètes comporte 96 régions hydrophiles.

13. Matériau selon l'une des revendications 1 à 12,
dans lequel
chaque région hydrophile discrète a une forme circulaire.

14. Matériau polymère poreux en une seule pièce présentant une surface comprenant un ensemble de régions hydrophiles discrètes revêtues de molécules polyélectrolytiques, ces molécules polyélectrolytiques comprenant un ensemble de groupes fonctionnels et étant reliées aux régions hydrophiles discrètes par un ensemble de liaisons covalentes et d'interactions électrostatiques.

15. Matériau selon la revendication 14,
dans lequel
l'ensemble de groupes fonctionnels présente des charges électriques.

16. Matériau selon la revendication 15,
dans lequel
les charges électriques comprennent des charges positives, des charges négatives, ou des combinaisons de telles charges.

17. Matériau selon l'une des revendications 14-16,
dans lequel
l'ensemble de groupes fonctionnels est choisi dans le groupe formé par les fonctions amine, acide carboxylique, sulfate, les polysaccharides, les agents tensioactifs, les agents tensioactifs fluorés et la biotine.

18. Matériau selon l'une des revendications 14-17,
dans lequel
les groupes fonctionnels sont identiques.

19. Matériau selon l'une des revendications 14-17,
dans lequel
les groupes fonctionnels sont différents.

20. Matériau selon l'une des revendications 14-19,
dans lequel
les molécules polyélectrolytiques comprennent les composés suivants : polyéthylèneimide, polyvinylimidazoline, polyacrylamide quaternisé, polyacrylamide modifié par des groupes carboxyle, polyvinylpyridine, polyvinylpyrrolidone, polyvinylamine, poly(allylamine), chitosane, polylysine, ester sel de polyacrylate trialkylammonium, cellulose, acide polyacrylique, acide méthylacrylique, acide polymaléique, acide polystyrène sulfonique, acide polyvinylsulfonique, acide polytoluène sulfonique, poly(méthyl vinyl éther-alt-acide-maléique), acide polyglutamique, agent tensioactif, sulfate de dextrane, acide hyaluronique, héparine, acide alginique, acide adipique, colorant chimique, protéine, enzyme, acide nucléique, peptide, ou leurs sels esters ou copolymères.

21. Procédé de préparation de matériaux poreux comprenant un ensemble de régions hydrophiles discrètes comprenant les étapes consistant à :
- masquer un substrat polymère poreux pour obtenir une surface à motifs comprenant des régions masquées et des régions non-masquées,
- activer les régions non-masquées, et
- revêtir les régions non-masquées activées avec une première couche de molécules polyélectrolytiques, ces molécules polyélectrolytiques étant liées aux régions non-masquées activées par des liaison covalentes et des interactions électrostatiques.

22. Procédé conforme à la revendication 21,
selon lequel
l'étape d'activation comporte l'exposition des régions non-masquées à une activation par plasma; une décharge de corona, un rayonnement électronique ou un rayonnement ultraviolet.

23. Procédé selon la revendication 21 ou 22,
comprenant en outre une étape consistant à revêtir les régions non-masquées activées avec une seconde couche de molécules polyélectrolytiques, cette seconde couche de molécules polyélectrolytiques étant liée à la première caliche par des liaisons covalentes et des interactions électrostatiques.

24. Procédé conforme à lune des revendications 21-23,
selon lequel
les molécules polyélectrolytiques de la première couche et/ou de la seconde couche, sont choisies indépendamment dans le groupe formé par les composés suivants :
polyéthylèneimide, polyvinylimidazoline, polyacrylamide quaternisé, polyacrylamide modifié par des groupes carboxyle, polyvinylpyridine, polyvinylpyrrolidone, polyvinylamine, poly(allylamine), chitosane, polylysine, ester sel de polyacrylate trialkyl ammonium, cellulose, acide polyacrylique, acide polyméthylacrylique, acide polymaléique, acide polystyrène sulfonique, acide polyvinyl sulfonique, acide polytoluène sulfonique, poly(méthyl vinyle éther-alt-acide-maléique), acide polyglutamique, agent tensioactif, sulfate de dextrane, acide hyaluronique, héparine, acide alginique, acide adipique, colorant chimique, protéine, enzyme, acide nucléique, peptide, ou leurs sels, esters ou copolymères.

25. Procédé selon la revendication 23 ou 24,
comportant en outre une étape consistant à revêtir les régions non-masquées activées avec au moins une couche supplémentaire, chacune de cette ou de ces couche(s) supplémentaire(s) étant liée(s) à la couche située en dessous par des liaisons covalentes, des interactions électrostatiques ou des combinaisons de celles-ci.

26. Procédé conforme à la revendication 25,
selon lequel
la ou les couche(s) supplémentaire(s) comporte(nt) un polyélectrolyte, un polymère neutre, une petite molécule, une biomolécule ou une combinaison de ces composés.

27. Procédé conforme à la revendication 26,
selon lequel
la biomolécule comporte une protéine, une enzyme, une hormone, un peptide, un acide nucléique, un oligonucléotide, un acide nucléique, ou un polysaccharide.

28. Procédé de préparation de matériaux poreux comprenant un ensemble de régions hydrophiles discrètes comprenant les étapes consistant à :
- se procurer un substrat poreux,
- disposer une poudre polymère selon un motif sur le substrat poreux, et
- fritter par compression le substrat poreux et la poudre.

29. Procédé conforme à la revendication 28,
selon lequel
le substrat est choisi parmi les composés suivants :
polyoléfine, polyéther, polyuréthane, polycarbonate, polyétheréthercétone, oxyde de polyphénylène, polyéther sulfone, polysulfone, nitrocellulose, cellulose, polymères fluorés, PTFE, matériau fibreux poreux ou nylon.

30. Procédé conforme à la revendication 29, selon lequel
la polyoléfine est choisie dans le groupe formé par les composés suivant : éthylène vinylacétate, éthylène méthylacrylate, polyéthylène, polypropylène, caoutchouc d'éthylène-propylène, caoutchoucs éthylène-propylène-diène, poly(1-butène), polystyrène, poly(2-butène), poly(1-pentène), poly(2-pentène), poly(3-méthyl-1-pentène), poly(4-méthyl-1-pentène), 1,2-poly-1,3-butadiène, 1,4-poly-1,3-butadiène, polyisoprène, polychloroprène, polyvinylacétate, chlorure de polyvinylidène, fluorure de polyvinylidène, polytétrafluoroéthylène, ou leurs mélanges.

31. Procédé conforme à l'une des revendications 28-30, selon lequel la poudre polymère comporte un ensemble de polymères différents.

32. Procédé selon l'une des revendications 28-31,
comportant en outre une étape consistant à revêtir l'ensemble de régions hydrophiles discrètes avec une première couche de molécules polyélectrolytiques, ces molécules polyélectrolytiques étant liées aux régions hydrophiles discrètes par des liaison covalentes et des interactions électrostatiques.

33. Procédé conforme à la revendication 32,
comprenant en outre une étape consistant à revêtir l'ensemble des régions hydrophiles discrètes avec une seconde couche de molécules polyélectrolytiques, la seconde couche de molécules polyélectrolytiques étant liée à la première couche par des liaison covalentes et des interactions électrostatiques.

34. Procédé conforme à la revendication 32 ou 33,
selon lequel
les molécules polyélectrolytiques de la première couche et/ ou de la seconde couche sont choisies indépendamment dans le groupe formé par les composés suivants : polyéthylèneimide, polyvinylimidazoline, polyacrylamide quaternisé, polyacrylamide modifié par des groupes carboxyle, polyvinylpyridine, polyvinylpyrrolidone, polyvinylamine, polyallylamine, chitosane, polylysine, ester sel de polyacrylate trialkyl ammonium, cellulose, acide polyacrylique, acide polyméthylacrylique, acide polymaléique, acide polystyrène sulfonique, acide polyvinylsulfonique, acide polytoluène sulfonique, poly(méthyl vinyl éther-alt-acide-maléique), acide polyglutamique, agent tensioactif, sulfate de dextrane, acide hyaluronique, héparine, acide alginique, acide adipique, colorant chimique, protéine, enzyme, acide nucléique, peptide, ou leurs sel esters ou copolymères.

35. Procédé conforme à la revendication 33 ou 34,
comportant en outre une étape consistant à revêtir l'ensemble des régions discrètes avec au moins une couche supplémentaire, cette ou chacune de ces couches supplémentaires étant reliée à la couche située en dessous par liaisons covalentes, des interactions électrostatiques ou des combinaisons de celles-ci.

36. Procédé conforme à la revendication 35,
selon lequel
la ou les couche(s) supplémentaire(s) comporte(nt) un polyélectrolyte, un polymère neutre, une petite molécule, une biomolécule ou leurs combinaisons.

37. Procédé conforme à la revendication 36,
selon lequel
la petite molécule comporte du dodécylsulfate de sodium, du bromure de dodécyltriméthylammonium, un phosphate, un sulfonate, un bromate, un colorant, un lipide, un ion métallique ou un agent tensioactif fluoré.

38. Procédé conforme à la revendication 36,
selon lequel
la biomolécule comporte une protéine, une enzyme, une hormone, un peptide, un acide nucléique, un oligonucléotide, ou un polysaccharide.
